# EUROPEAN PATENT APPLICATION

(11) **EP 4 331 571 A1**
(43) Date of publication of application: **06.03.2024**
(21) Application number: 23193886.1
(22) Date of filing: 29.08.2023
(51) Int. Cl.: A61K 9/00, A61K 39/12, A61P 31/14, C07K 16/10, A61K 47/12, A61K 47/26

(54) **FORMULATIONS OF ACE2-IGM FUSION PROTEINS**

(30) Priority: 02.09.2022 EP 22193790
(71) Applicant: Formycon AG, 82152 Planegg (DE)
(72) Inventor: SIGL, Rainer, 86899 Landsberg am Lech (DE)
(74) Representative: Maiwald GmbH

(57) **Abstract**

The present invention relates to pharmaceutical compositions of ACE2-lgM fusion proteins and therapeutic uses thereof, in particular in the treatment of infections with SARS-CoV-2.

## Description

### FIELD OF THE INVENTION

The present invention relates to pharmaceutical compositions of ACE2-lgM fusion proteins and therapeutic uses thereof, in particular in the treatment of infections with SARS-CoV-2.

### BACKGROUND OF THE INVENTION

ACE2 (angiotensin converting enzyme 2) is a key metalloprotease of the renin-angiotensin system with a catalytic zinc atom in the centre (Donoghue et al. (2002) Circ. Res. 87: e1-e9). Full-length ACE2 consists of an N-terminal extracellular peptidase domain, a collectrin-like domain, a single transmembrane helix and a short intracellular segment. It acts to cleave Angiotensin II to produce Angiotensin (1-7) and Angiotensin I to produce Angiotensin (1-9) which is then processed by other enzymes to become Angiotensin (1-7). ACE2 acts to lower the blood pressure and counters the activity of ACE in order to maintain a balance in the Ras/MAS system. Accordingly, it is a promising target for the treatment of cardiovascular diseases.

Recently, ACE2 has gained much attention as a receptor for coronaviruses and in particular the novel coronavirus SARS-CoV-2. SARS-CoV-2 is a coronavirus which was first discovered in December 2019 in Wuhan, China, but spread rapidly all over the world, leading to a world-wide pandemic. The pandemic led to a lock-down in many countries with very significant economic and social effects.

It was shown that ACE2 functions as a receptor for SARS-CoV (Li et al. (2003) Nature 426: 450-454; Prakabaran et al. (2004) Biochem. Biophys. Res. Comm. 314: 235-241) and for SARS-CoV-2 (Yan et al. (2020) Science 367: 1444-1485). Further, entry of SARS-CoV-2 into the respiratory cells depends on ACE2 and the serine protease TMPRSS2 (Hoffmann et al. (2020) Cell 181: 1-10).

In view of the important role of ACE2 for virus entry into the cell, it was proposed to use soluble ACE2 for blocking SARS binding to the cell (WO 2005/032487; WO 2006/122819). The same approach was also suggested for the treatment of infections with SARS-CoV-2 (Kruse (2020) F1000Res. 9:72). Clinical trials with a soluble form of ACE2 in the treatment of infections with SARS-CoV-2 have been initiated by the company Apeiron (Pharmazeutische Zeitung, 10 April 2020) and the first results showed that one patient with severe Covid-19 caused by SARS-CoV-2 recovered fast upon treatment with soluble ACE2 (Zoufaly et al. (2020) The Lancet Respiratory Medicine 8: 1154-1158).

However, isolated receptor domains are typically characterized by a low stability and plasma half-life. For the soluble form of ACE2 a dose-dependent terminal half-life of 10 hours was shown (Haschke et al. (2013) Clin. Pharmacokinet. 52: 783-792). In view of these results, it was decided to administer the soluble form of ACE2 as twice-daily infusion in a later study (Khan et al. (2017) Critical Care 21: 234). However, an administration of more than one time per day is inconvenient both for the patient and the medical personnel.

A fusion protein of ACE2 consisting of the extracellular domain of either enzymatically active or enzymatically inactive ACE2 linked to the Fc domain of human IgG1 was constructed and tested. It was shown that both constructs potently neutralized both SARS-CoV and SARS-CoV-2 and inhibited S protein-mediated fusion (Lei et al. (2020) Nature Communications 11: 2070). Liu et al. (2020) Int. J. Biol. Macromol. 165: 1626-1633 describe fusion proteins of wild-type ACE2 and nine ACE2 mutants affecting catalytic activity of ACE2 with the Fc region of human IgG1. However, the interaction of the Fc domain of human IgG1 with Fc gamma receptors on immune cells may enhance the virus infection (Perlman and Dandekar (2005) Nat. Rev. Immunol. 5(12): 917-927; Chen et al. (2020) Current Tropical Medicine Reports 3:1-4).

Tada et al. (2020), available at https://www.biorxiv.org/content/10.1101/2020.09.16.300319v1.full, disclose an "ACE2 microbody" in which the extracellular domain of catalytically inactive ACE2 is fused to the Fc domain of the immunoglobulin heavy chain.

Svilenov et al. (2020), available at Efficient inhibition of SARS-CoV-2 strains by a novel 10 ACE2-lgG4-Fc fusion protein with a stabilized hinge region I bioRxiv and https://www.sciencedirect.com/science/article/pii/S016635422100187X7via%3Dihub, International patent applications WO 2021/234160 A2 and WO2022/090469 are directed to fusion proteins of the extracellular domain of ACE2 with the Fc domain of human IgG1 or IgG4. European patent application EP 21 210 215.6 is directed to highly sialylated and/or afucosylated forms of fusion proteins of the extracellular domain of ACE2 with the Fc domain of human IgG1 or IgG4 as well as to such fusion proteins which additionally comprise IP10.

International patent application PCT/EP2022/071308 discloses ACE2 fusion proteins with the Fc domain of IgM and IgG2.

WO 2021/170113, WO 2021/183404, US 2021/0284716, WO 2021/217120, WO 2021/213437, WO 2021/189772, WO 2021/231466, WO 2021/237239, WO 2021/203103, WO 2021/174107, WO 2021/236957, WO 2021/203098, WO 2021/227937, WO 2021/213421, WO 2021/205183, WO 2021/202427, WO 2021/194909, WO 2021/170131, WO 2022/012688, WO 2022/006601, WO 2021/263128 and WO 2021/257512 also disclose fusion proteins of ACE2 with the Fc part of different immunoglobulins.

There is a need for pharmaceutical compositions of ACE2 Fc fusion proteins which provide the required protein stability during storage and which are well tolerated by human patients.

In clinical trials using soluble recombinant human ACE2 a formulation comprising 5.2 mg/ml recombinant human ACE2 in 100 mM glycine, 150 mM NaCl and 50 µM zinc chloride at a pH of 7.5 was used (Haschke et al. (2013) Clin. Pharmacokinet. 52: 783-792). International patent application PCT/EP2022/055457 is directed to pharmaceutical compositions comprising fusion proteins of ACE2 with the Fc part of human IgG and a buffer.

### SUMMARY OF THE INVENTION

The present invention provides stable liquid formulations of ACE2 Fc fusion proteins which are suitable for administration to human subjects.

The present invention provides a liquid pharmaceutical composition comprising:
(a) a fusion protein comprising a first part comprising a fragment of human ACE2 or a variant of said fragment, said human ACE2 having the amino acid sequence according to SEQ ID NO: 1, and a second part comprising the Fc portion of a human IgM or a fragment or variant of the Fc portion of human IgM; and
(b) a buffer having a pH of 5.4 to 6.4.

In one embodiment, the buffer is selected from the group consisting of acetate buffer, histidine buffer, phosphate buffer, citrate buffer, and succinate buffer, preferably wherein the buffer is present in a concentration of 5 mM to 60 mM.

The liquid pharmaceutical composition may further comprise a sugar or a sugar alcohol, which may be selected from trehalose, sucrose, sorbitol and mannitol and/or which may be present in a concentration of 100 mM to 300 mM.

The liquid pharmaceutical composition may further comprise a non-ionic surfactant, which may be selected from polysorbate 20 and polysorbate 80 and/or which may be present in a concentration of 0.01 % (w/v) to 0.2% (w/v).

The liquid pharmaceutical composition may further comprise an inorganic salt, preferably which may be sodium chloride and/or which may be present in a concentration of 30 mM to 150 mM.

The liquid pharmaceutical composition may further comprise one or more amino acids, which which may be present in a concentration of 1 mM to 50 mM.

In one embodiment, the fragment of human ACE2 consists of the amino acid sequence according to SEQ ID NO: 2 or the fragment of human ACE2 is the extracellular domain of ACE2 consisting of the amino acid sequence according to SEQ ID NO: 3.

In one embodiment, the Fc portion of human IgM has the amino acid sequence according to SEQ ID NO: 4 or the fragment of the Fc portion of human IgM consists of the amino acid sequence according to SEQ ID NO: 7.

In one embodiment, the fusion protein has the amino acid sequence according to SEQ ID NO: 8 or 9.

In one embodiment, the variant of the human ACE2 fragment is an enzymatically inactive variant of human ACE2 which may comprise a H374N and a H378N mutation, the numbering referring to SEQ ID NO: 1.

In one embodiment, the fusion protein is present as a multimer formed by at least two molecules of said fusion protein.

The present invention further relates to a liquid pharmaceutical composition comprising:
(a) a fusion protein comprising a first part comprising a fragment of human ACE2 or a variant of said fragment, said human ACE2 having the amino acid sequence according to SEQ ID NO: 1, and a second part comprising the Fc portion of a human IgM or a fragment or variant of the Fc portion of human IgM; and
(b) an acetate buffer having a pH of 5.6 to 5.8;
(c) polysorbate 20 or polysorbate 80;
(d) trehalose or sucrose; and
(e) optionally, one or more stabilizers selected from the group consisting of L-arginine, L-methionine and sodium chloride.

In one embodiment, the fusion protein is present in a concentration of 1-60 mg/ml.

The present invention also relates to said liquid pharmaceutical composition for use in preventing and/or treating an infection with a coronavirus binding to ACE2, preferably wherein the coronavirus binding to ACE2 is selected from the group consisting of SARS, SARS-CoV2 and NL63, preferably it is SARS-CoV2.

### DETAILED DESCRIPTION OF THE INVENTION

The present invention as illustratively described in the following may suitably be practiced in the absence of any element or elements, limitation or limitations, not specifically disclosed herein.

The present invention will be described with respect to particular embodiments, but the invention is not limited thereto, but only by the claims.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. a cell or organism is defined to be obtainable by a specific method, this is also to be understood to disclose a cell or organism which is obtained by this method.

Where an indefinite or definite article is used when referring to a singular noun, e.g. "a", "an" or "the", this includes a plural of that noun unless something else is specifically stated.

The term "pharmaceutical composition" as used herein refers to any composition comprising a chemical substance or active ingredient such as the ACE2 Fc fusion protein which composition is intended for use in the medical cure, treatment, or prevention of disease and which is in such a form as to permit the active ingredient to be effective. In particular, a pharmaceutical composition does not contain excipients which are unacceptably toxic to a subject to which the composition is to be administered. The pharmaceutical compositions are sterile, i.e. aseptic and free from all living microorganisms and their spores. The pharmaceutical composition used in the present invention is liquid and stable.

In a "liquid pharmaceutical composition" the pharmaceutically active agent, e.g. the ACE2 Fc fusion protein, can be combined with a variety of excipients to ensure a stable active medication following storage. In one embodiment, the liquid pharmaceutical composition used in the invention is at no point lyophilized, i.e. the production method does not contain a lyophilization step and the composition is not lyophilized for storage. Liquid pharmaceutical compositions can be stored in vials, IV bags, ampoules, cartridges, and prefilled or ready-to-use syringes.

A "stable" liquid composition is one in which the ACE2 Fc fusion protein contained therein essentially retains its physical stability and/or chemical stability and/or biological activity upon storage for a certain period. Preferably, the composition essentially retains upon storage its physical and chemical stability, as well as its biological activity. Various analytical techniques for measuring protein stability are available in the art and are reviewed, for example, in Peptide and Protein Drug Delivery, 247-301, Vincent Lee Ed, Marcel Dekker, Inc, New York, New York, Pubs (1991) and Jones, Adv Drug Delivery Rev, 1993, 10:29-90. For example, stability can be measured at a selected temperature for a selected time period. Stability can be evaluated qualitatively and/or quantitatively in a variety of different ways, including evaluation of aggregate formation (for example using size exclusion chromatography, by measuring turbidity, and/or by visual inspection), by assessing charge heterogeneity using cation or anion exchange chromatography or capillary zone electrophoresis, amino-terminal or carboxy-terminal sequence analysis, mass spectrometric analysis, SDS-Capillary gel electrophoresis analysis to detect aggregated or fragmented molecules, peptide map (for example tryptic or LYS-C) analysis, evaluating biological activity or binding of the agonist, etc.

Preferably, the pharmaceutical composition is stable at a temperature of about 40°C and 75% relative humidity for at least 1 to 2 weeks, and/or is stable at a temperature of about 5°C for at least 3 months, preferably 6 or 8 months and more preferably one year, and/or is stable at a temperature of about 25°C and 60% relative humidity for at least two weeks or one month. Furthermore, the formulation is preferably stable following freezing (to, e.g., -20°C or -80°C) and thawing of the formulation at 25°C as described in the examples herein, for example following 1, 2, 3 or 4 cycles of freezing and thawing.

For example, in the pharmaceutical composition used in the present invention the absolute increase of the percentage of high molecular weight species in the sample after storage for 3 months at a temperature of 5°C compared to the percentage of high molecular weight species in the sample before storage for 3 months at a temperature of 5°C as measured by size exclusion chromatography is not more than 10 percentage points, preferably not more than 8 percentage points and more preferably not more than 5 percentage points. For example, if the percentage of high molecular weight species in the sample before storage for 3 months at a temperature of 5°C was 0.5%, the percentage of high molecular weight species in the sample after storage for 3 months at a temperature of 5°C is not more than 10.5%, preferably not more than 8.5% and more preferably not more than 5.5%.

A "buffer" is an aqueous solution consisting of a mixture of a weak acid and its conjugate base or vice versa which resists changes in pH and therefore keeps the pH at a nearly constant value. The buffer used in the present invention has a pH in the range from about 5.4 to about 6.4. Preferably, the buffer used in the present invention has a pH in the range from about 5.4 to about 6.2, more preferably in the range from about 5.4 to 6.0 and most preferably in the range from 5.4 to 5.9 or 5.6 to 5.8. The buffer used in the present invention has a pH of 5.4, 5.5, 5.6, 5.7, 5.8, 5.9, 6.0, 6.1, 6.2, 6.3 or 6.4, preferably of 5.6 or 5.8.

The buffer used in the present invention is selected from the group consisting of an acetate buffer, a histidine buffer, a citrate buffer, a phosphate buffer and a succinate buffer. Preferably, the liquid pharmaceutical composition of the present invention comprises only one buffer type, more preferably it comprises only one buffer type selected from an acetate buffer, a histidine buffer, a citrate buffer, a phosphate buffer and a succinate buffer. Most preferably, the the liquid pharmaceutical composition of the present invention comprises an acetate buffer.

The acetate buffer may be prepared by mixing glacial acetic acid with an acetate salt such as sodium acetate. The acetate buffer has a concentration of 1 mM to 50 mM, preferably of 3 mM to 40 mM, more preferably of 5 mM to 30 mM, even more preferably of 7 mM to 20 mM and most preferably of 10 mM. Preferably, the acetate buffer has a pH in the range from about 5.2 to 6.0, preferably from about 5.3 to 5.9 or 5.4 to 5.8, and most preferably has a pH of about 5.4 or 5.8. Preferably, the acetate buffer has a pH of 5.4, 5.5, 5.6, 5.7 or 5.8.

In one embodiment, the acetate buffer has a concentration of 10 mM. In another embodiment the acetate buffer has a concentration of 10 mM and a pH of 54 or 5.8.

In one embodiment, the acetate buffer comprises glacial acetic acid and sodium acetate in a concentration of 10 mM. In another embodiment the acetate buffer comprises glacial acetic acid and sodium acetate in a concentration of 10 mM and with a pH of 5.4 or 5.8.

A "surfactant" as used herein refers to an amphiphilic compound, i.e. a compound containing both hydrophobic groups and hydrophilic groups which lowers the surface tension (or interfacial tension) between two liquids or between a liquid and a solid. A "non-ionic surfactant" has no charged groups in its head. The formation of insoluble particles during freeze/thaw cycles of protein-containing compositions can be remarkably inhibited by addition of surfactants. Examples of "non-ionic surfactants" include e.g. polyoxyethylene glycol alkyl ethers, such as octaethylene glycol monododecyl ether, pentaethylene glycol monododecyl ether; polyoxypropylene glycol alkyl ethers; glucoside alkyl ethers, such as decyl glucoside, lauryl glucoside, octyl glucoside; polyoxyethylene glycol octylphenol ethers, such as triton X-100; polyoxyethylene glycol alkylphenol ethers, such as nonoxynol-9; glycerol alkyl esters, such as glyceryl laurate; polyoxyethylene glycol sorbitan alkyl esters, such as polysorbate; sorbitan alkyl esters, such as spans; cocamide MEA, cocamide DEA, dodecyldimethylamine oxide; block copolymers of polyethylene glycol and polypropylene glycol, such as poloxamers; and polyethoxylated tallow amine (POEA). The liquid pharmaceutical composition of the present invention can contain one or more of these surfactants in combination. In a preferred embodiment the liquid pharmaceutical composition of the present invention comprises only one non-ionic surfactant.

Preferred non-ionic surfactants for use in the liquid pharmaceutical composition of the present invention are polysorbates such as polysorbate 20, 40, 60 or 80, and especially polysorbate 20 (i.e. Tween 20) or polysorbate 80 (i.e. Tween 80). Another preferred non-ionic surfactant for use in the pharmaceutical compositions of the present invention is poloxamer 188.

The concentration of the non-ionic surfactant is in the range of 0.005 to 0.20% (w/v), preferably in the range of 0.01 to 0.10 % (w/v), and most preferably in the range of 0.02 to 0.10% (w/v), relative to the total volume of the composition.

In a preferred embodiment, the non-ionic surfactant is polysorbate 20. In a preferred embodiment, the non-ionic surfactant is polysorbate 20 with a concentration in the range of 0.005% to 0.20% (w/v), preferably in the range of 0.008 to 0.05% (w/v), and most preferably in the range of 0.01 to 0.04% (w/v), relative to the total volume of the composition.

In one embodiment, the non-ionic surfactant is polysorbate 20 with a concentration of 0.02% (w/v).

In one embodiment, the buffer is an acetate buffer with a concentration of 10 mM and a pH of 5.4 and the surfactant is polysorbate 20 with a concentration of 0.02% (w/v).

In one embodiment, the buffer is an acetate buffer with a concentration of 10 mM and a pH of 5.8 and the surfactant is polysorbate 20 with a concentration of 0.02% (w/v).

The term "sugar" refers to an organic compound comprising only carbon, hydrogen, and oxygen, usually with a hydrogen:oxygen atom ratio of 2:1 and the empirical formula Cm(H2O)n. The term "sugar" includes mono-, di-, oligo- and polysaccharides. Examples of sugars include glucose, fructose, galactose, xylose, ribose, sucrose, mannose, lactose, maltose, trehalose, starch, and glycogen. Preferably, the sugar is a non-reducing sugar. Non-reducing sugars are sugars which are not able to act as a reducing agent, as they do not comprise a free aldehyde or ketone group. Preferably, the non-reducing sugar is selected from sucrose and trehalose.

In one embodiment, the sugar is trehalose. Trehalose is a non-reducing sugar. It is a disaccharide formed by a 1,1-glycosidic bond between a glucose and a fructose unit. Preferably, the dihydrate form of trehalose is used. The terms "trehalose" and " trehalose dihydrate" are used interchangeably herein. The concentration of trehalose dihydrate in the liquid pharmaceutical composition of the present invention is 100 mM to 300 mM, preferably the concentration of trehalose dihydrate is 100 mM to 250 mM, more preferably the concentration of trehalose dihydrate is 106 mM to 250 mM, even more preferably the concentration of trehalose dihydrate is 106 mM, 220 mM or 250 mM and most preferably the concentration of trehalose dihydrate is 250 mM.

In one embodiment, the buffer is an acetate buffer with a concentration of 10 mM and a pH of 5.8 and the sugar is trehalose with a concentration of 250 mM.

In one embodiment, the buffer is an acetate buffer with a concentration of 10 mM and a pH of 5.8, the sugar is trehalose with a concentration of 250 mM and the surfactant is polysorbate 20 with a concentration of 0.02% (w/v).

In one embodiment, the buffer is an acetate buffer with a concentration of 10 mM and a pH of 5.4 and the sugar is trehalose with a concentration of 250 mM.

In one embodiment, the buffer is an acetate buffer with a concentration of 10 mM and a pH of 5.4, the sugar is trehalose with a concentration of 250 mM and the surfactant is polysorbate 20 with a concentration of 0.02% (w/v).

In one embodiment the liquid pharmaceutical composition of the present invention comprises a sugar alcohol. Sugar alcohols are organic compounds derived from a sugar which contain a hydroxyl group attached to each carbon atom. Suitable sugar alcohols include glycerol, mannitol, sorbitol, and xylitol. The concentration of the sugar alcohol in the liquid pharmaceutical composition of the present invention is 50 mM to 300 mM, preferably the concentration of the sugar alcohol is 80 mM to 280 mM, more preferably the concentration of the sugar alcohol is 80 mM to 270 mM and most preferably the concentration of the sugar alcohol is 80 mM to 270 mM.

Preferably, the sugar alcohol is mannitol or sorbitol and more preferably the sugar alcohol is mannitol. The concentration of mannitol or sorbitol in the liquid pharmaceutical composition of the present invention is 50 mM to 300 mM, preferably the concentration of mannitol or sorbitol is 80 mM to 280 mM, more preferably the concentration of mannitol or sorbitol is 80 mM to 270 mM and most preferably the concentration of mannitol or sorbitol is 80 mM to 270 mM.

The concentration of mannitol in the liquid pharmaceutical composition of the present invention is 50 mM to 300 mM, preferably the concentration of mannitol is 80 mM to 280 mM, more preferably the concentration of mannitol is 80 mM to 270 mM and most preferably the concentration of mannitol is 80 mM to 270 mM.

In one embodiment the liquid pharmaceutical composition of the present invention comprises 88.5 mM, 198 mM, 225 mM or 252 mM sorbitol.

In one embodiment the liquid pharmaceutical composition of the present invention comprises 50 mM mannitol.

In one embodiment, the liquid pharmaceutical composition of the present invention comprises an inorganic salt. As used herein, an "inorganic salt" refers to an ionic compound which has osmoregulatory properties. An inorganic salt such as sodium chloride (NaCl) can dissociate in solution into its constituent ions, i.e. NaCl dissociates into Na+ and Cl- ions, which both affect the osmotic pressure, i.e. the osmolality, of the solution. Exemplary inorganic salts which may be present in the liquid pharmaceutical composition of the present invention are potassium chloride, calcium chloride, sodium chloride, sodium phosphate, potassium phosphate and sodium bicarbonate. In one embodiment, liquid pharmaceutical composition of the present invention comprises sodium chloride.

In one embodiment, the inorganic salt is present in a concentration of 20 to 150 mM, preferably in a concentration of 30 to 140 mM and more preferably in a concentration of 40 mM to 135 mM.

In one embodiment, the inorganic salt is sodium chloride and the sodium chloride is present in a concentration of 20 to 150 mM, preferably in a concentration of 30 to 140 mM and more preferably in a concentration of 40 mM to 135 mM. In one embodiment, the sodium chloride is present in a concentration of 40 mM to 50 mM, preferably in a concentration of 50 mM.

In one embodiment, the buffer is an acetate buffer with a concentration of 10 mM and a pH of 5.8 and the inorganic salt is sodium chloride with a concentration of 50 mM.

In one embodiment, the buffer is an acetate buffer with a concentration of 10 mM and a pH of 5.4 and the inorganic salt is sodium chloride with a concentration of 50 mM.

In one embodiment, the buffer is an acetate buffer with a concentration of 10 mM and a pH of 5.8, the surfactant is polysorbate 20 with a concentration of 0.02% (w/v) and the inorganic salt is sodium chloride with a concentration of 50 mM.

In one embodiment, the buffer is an acetate buffer with a concentration of 10 mM and a pH of 5.4, the surfactant is polysorbate 20 with a concentration of 0.02% (w/v) and the inorganic salt is sodium chloride with a concentration of 50 mM.

In one embodiment, the buffer is an acetate buffer with a concentration of 10 mM and a pH of 5.8, the surfactant is polysorbate 20 with a concentration of 0.02% (w/v), the sugar is trehalose with a concentration of 250 mM and the inorganic salt is sodium chloride with a concentration of 50 mM.

In one embodiment, the buffer is an acetate buffer with a concentration of 10 mM and a pH of 5.4, the surfactant is polysorbate 20 with a concentration of 0.02% (w/v), the sugar is trehalose with a concentration of 250 mM and the inorganic salt is sodium chloride with a concentration of 50 mM.

In one embodiment, the liquid pharmaceutical composition of the present invention comprises a salt of a transition metal such as salts of zinc, copper and manganese. Exemplary salts of transition metals which may be present in the liquid pharmaceutical composition of the present invention include zinc chloride, zinc sulfate, copper chloride, copper sulfate, manganese chloride and manganese sulfate. In a preferred embodiment, the liquid pharmaceutical composition of the present invention comprises a zinc salt such as zinc chloride or zinc sulfate.

In one embodiment, the salt of a transition metal is zinc chloride and the zinc chloride is present in a concentration of 10 µM to 300 µM, preferably of 20 µM to 250 µM, more preferably of 30 µM to 220 µM and most preferably of 50 µM to 200 µM.

In one embodiment, the salt of a transition metal is zinc chloride and the concentration of the zinc ion is adjusted dependent on the concentration of the fusion protein present in the liquid pharmaceutical composition of the present invention. In one embodiment, the stoichometric ratio between the amount of the fusion protein and the amount of the zinc ion is 1:2, i.e. two zinc ions per protein molecule are present in the liquid pharmaceutical composition of the present invention. In one example, the concentration of the fusion protein is 20 mg/ml and the concentration of the zinc ions is 200 µM.

In one embodiment, the liquid pharmaceutical composition of the present invention comprises one or more amino acids. In one embodiment the one or more amino acids are selected from the group of proteinogenic amino acids. In one embodiment, the one or more amino acids are selected from the group consisting of L-arginine, glycine, L-methionine, L-asparagine, L-glutamine, L-lysine, L-alanine, L-leucine, L-isoleucine, L-glutamic acid, L-aspartic acid and L-proline. In one embodiment, the liquid pharmaceutical composition of the present invention comprises L-arginine and/or L-methionine, preferably the liquid pharmaceutical composition of the present invention comprises L-arginine and L-methionine. In one embodiment, the liquid pharmaceutical composition of the present invention does not comprise glycine. In one embodiment, the liquid pharmaceutical composition of the present invention does not comprise 100 mM glycine.

In one embodiment, the one or more amino acids is/are present in a concentration of 1 mM to 150 mM, preferably in a concentration of 2 mM to 140 mM and more preferably in a concentration of 5 mM to 135 mM.

In one embodiment, the liquid pharmaceutical composition of the present invention comprises L-arginine in a concentration of 20 mM to 60 mM, preferably of 25 mM to 55 mM, more preferably of 30 mM to 50 mM and most preferably of 30 mM. In one embodiment, the liquid pharmaceutical composition of the present invention comprises L-arginine in a concentration of 30 mM, 45 mM or 50 mM, preferably of 30 mM.

In one embodiment, the liquid pharmaceutical composition of the present invention comprises L-methionine in a concentration of 1 mM to 20 mM, preferably of 3 mM to 15 mM, more preferably of 5 mM to 10 mM and most preferably of 5 mM. In one embodiment, the liquid pharmaceutical composition of the present invention comprises L-methionine in a concentration of 5 mM.

In one embodiment, the liquid pharmaceutical composition of the present invention comprises L-methionine in a concentration of 5 mM and L-arginine in a concentration of 30 mM.

In one embodiment, the liquid pharmaceutical composition of the present invention comprises an acetate buffer with a concentration of 10 mM and a pH of 5.8 and 5 mM L-methionine.

In one embodiment, the liquid pharmaceutical composition of the present invention comprises an acetate buffer with a concentration of 10 mM and a pH of 5.8 and 30 mM L-arginine.

In one embodiment, the liquid pharmaceutical composition of the present invention comprises an acetate buffer in a concentration of 10 mM and with a pH of 5.8, 5 mM L-methionine and 30 mM L-arginine.

In one embodiment, the liquid pharmaceutical composition of the present invention comprises an acetate buffer in a concentration of 10 mM and with a pH of 5.8, polysorbate 20 in a concentration of 0.02% (w/v), 5 mM L-methionine and 30 mM L-arginine.

In one embodiment, the liquid pharmaceutical composition of the present invention comprises an acetate buffer in a concentration of 10 mM and with a pH of 5.8, polysorbate 20 in a concentration of 0.02% (w/v), trehalose in a concentration of 250 mM, 5 mM L-methionine and 30 mM L-arginine.

In one embodiment, the liquid pharmaceutical composition of the present invention comprises an acetate buffer with a concentration of 10 mM and a pH of 5.4 and 5 mM L-methionine.

In one embodiment, the liquid pharmaceutical composition of the present invention comprises an acetate buffer with a concentration of 10 mM and a pH of 5.4 and 30 mM L-arginine.

In one embodiment, the liquid pharmaceutical composition of the present invention comprises an acetate buffer in a concentration of 10 mM and with a pH of 5.4, 5 mM L-methionine and 30 mM L-arginine.

In one embodiment, the liquid pharmaceutical composition of the present invention comprises an acetate buffer in a concentration of 10 mM and with a pH of 5.4, polysorbate 20 in a concentration of 0.02% (w/v), 5 mM L-methionine and 30 mM L-arginine.

In one embodiment, the liquid pharmaceutical composition of the present invention comprises an acetate buffer in a concentration of 10 mM and with a pH of 5.4, polysorbate 20 in a concentration of 0.02% (w/v), trehalose in a concentration of 250 mM, 5 mM L-methionine and 30 mM L-arginine.

A "fusion protein" is a protein which is formed by at least two polypeptide parts which are not naturally linked with each other. The two polypeptide parts are linked by a peptide bond and optionally a linker molecule is inserted between the two polypeptide parts. The two polypeptide parts are transcribed and translated as a single molecule. The fusion protein typically has functionalities derived from both polypeptide parts. In the context of the present invention, the fusion protein retains the binding properties of ACE2, in particular the binding of viruses such as coronaviruses, and the increased half-life and Fc receptor binding conferred by the Fc portion of human IgM.

The term "human ACE2" refers to angiotensin converting enzyme 2 derived from a human subject. The full-length sequence of human ACE2 has 805 amino acids. It comprises a signal peptide, an N-terminal extracellular peptidase domain followed by a collectrin-like domain, a single transmembrane helix and a short intracellular segment. The full-length sequence of human ACE2 is depicted in SEQ ID NO: 1. Unless indicated otherwise, the amino acid numbering used herein refers to the numbering of the full-length sequence of human ACE2 according to SEQ ID NO: 1. The extracellular domain of human ACE2 consists of the amino acids 18 to 740 of SEQ ID NO: 1 and is shown in SEQ ID NO: 3. The signal peptide is depicted in SEQ ID NO: 6.

The term "fragment of human ACE2" refers to a polypeptide which lacks one or more amino acids compared to the full-length sequence of human ACE2 according to SEQ ID NO:1. The fragment of human ACE2 is capable of binding to the S protein of at least one coronavirus, in particular to the S protein of SARS-CoV-2. The binding of a fragment of human ACE2 to the S protein of at least one coronavirus can be determined in an ELISA assay in which the S protein is immobilized on a substrate and contacted with the fragment of human ACE2 and the interaction between the S protein and the fragment of human ACE2 is detected. Alternatively, the binding of a fragment of human ACE2 to the S protein of at least one coronavirus can be determined by surface plasmon resonance, e.g. as described in Shang et al. (2020) Nature doi: 10.1038/s41586-020-2179-y; Wrapp et al. (2020) Science 367(6483): 1260-1263; Lei et al. (2020) Nature Communications 11(1): 2070. In a further alternative, the binding of a fragment of human ACE2 to the S protein of at least one coronavirus can be determined by biolayer interferometry, e.g. as described in Seydoux et al. (2020) https://doi.org/10.1101/2020.05.12.091298.

In one embodiment, the fragment of human ACE2 consists of 360 to 723 contiguous amino acids within the sequence according to SEQ ID NO: 1. Preferably, the fragment of human ACE2 consists of 380 to 723, 400 to 723, 420 to 723, 440 to 723, 460 to 723, 480 to 723 or 500 to 723 contiguous amino acids within the sequence according to SEQ ID NO: 1. Preferably, the fragment of human ACE2 consists of 520 to 723, 540 to 723, 560 to 723, 580 to 723 or 600 to 723 contiguous amino acids within the sequence according to SEQ ID NO: 1. More preferably, the fragment of human ACE2 consists of 620 to 723, 640 to 723, 660 to 723, 680 to 723, 700 to 723 or 720 to 723 contiguous amino acids within the sequence according to SEQ ID NO: 1.

In one embodiment, the fragment of human ACE2 comprises the amino acid residues K31 and K353, the numbering referring to SEQ ID NO: 1. In one embodiment, the fragment of human ACE2 comprises the amino acid residues Q24, D30, E35 and Q42, the numbering referring to SEQ ID NO:1. In one embodiment, the fragment of human ACE2 comprises the amino acid residues Q24, D30, K31, E35, Q42 and K353, the numbering referring to SEQ ID NO: 1.

In one embodiment, the fragment of human ACE2 consists of 360 to 723 contiguous amino acids within the sequence according to SEQ ID NO: 1 and comprises the amino acid residues K31 and K353, the numbering referring to SEQ ID NO: 1. Preferably, the fragment of human ACE2 consists of 380 to 723, 400 to 723, 420 to 723, 440 to 723, 460 to 723, 480 to 723 or 500 to 723 contiguous amino acids within the sequence according to SEQ ID NO: 1 and comprises the amino acid residues K31 and K353, the numbering referring to SEQ ID NO: 1. Preferably, the fragment of human ACE2 consists of 520 to 723, 540 to 723, 560 to 723, 580 to 723 or 600 to 723 contiguous amino acids within the sequence according to SEQ ID NO: 1 and comprises the amino acid residues K31 and K353, the numbering referring to SEQ ID NO: 1. More preferably, the fragment of human ACE2 consists of 620 to 723, 640 to 723, 660 to 723, 680 to 723, 700 to 723 or 720 to 723 contiguous amino acids within the sequence according to SEQ ID NO: 1 and comprises the amino acid residues K31 and K353, the numbering referring to SEQ ID NO: 1.

In one embodiment, the fragment of human ACE2 consists of 360 to 723 contiguous amino acids within the sequence according to SEQ ID NO: 1 and comprises the amino acid residues Q24, D30, E35 and Q42, the numbering referring to SEQ ID NO: 1. Preferably, the fragment of human ACE2 consists of 380 to 723, 400 to 723, 420 to 723, 440 to 723, 460 to 723, 480 to 723 or 500 to 723 contiguous amino acids within the sequence according to SEQ ID NO: 1 and comprises the amino acid residues Q24, D30, E35 and Q42, the numbering referring to SEQ ID NO: 1. Preferably, the fragment of human ACE2 consists of 520 to 723, 540 to 723, 560 to 723, 580 to 723 or 600 to 723 contiguous amino acids within the sequence according to SEQ ID NO: 1 and comprises the amino acid residues Q24, D30, E35 and Q42, the numbering referring to SEQ ID NO: 1. More preferably, the fragment of human ACE2 consists of 620 to 723, 640 to 723, 660 to 723, 680 to 723, 700 to 723 or 720 to 723 contiguous amino acids within the sequence according to SEQ ID NO: 1 and comprises the amino acid residues Q24, D30, E35 and Q42, the numbering referring to SEQ ID NO: 1.

In one embodiment, the fragment of human ACE2 consists of 360 to 723 contiguous amino acids within the sequence according to SEQ ID NO: 1 and comprises the amino acid residues Q24, D30, K31, E35, Q42 and K353, the numbering referring to SEQ ID NO: 1. Preferably, the fragment of human ACE2 consists of 380 to 723, 400 to 723, 420 to 723, 440 to 723, 460 to 723, 480 to 723 or 500 to 723 contiguous amino acids within the sequence according to SEQ ID NO: 1 and comprises the amino acid residues Q24, D30, K31, E35, Q42 and K353, the numbering referring to SEQ ID NO: 1. Preferably, the fragment of human ACE2 consists of 520 to 723, 540 to 723, 560 to 723, 580 to 723 or 600 to 723 contiguous amino acids within the sequence according to SEQ ID NO: 1 and comprises the amino acid residues Q24, D30, K31, E35, Q42 and K353, the numbering referring to SEQ ID NO: 1. More preferably, the fragment of human ACE2 consists of 620 to 723, 640 to 723, 660 to 723, 680 to 723, 700 to 723 or 720 to 723 contiguous amino acids within the sequence according to SEQ ID NO: 1 and comprises the amino acid residues Q24, D30, K31, E35, Q42 and K353, the numbering referring to SEQ ID NO: 1.

In one embodiment, the fragment of human ACE2 consists of amino acids 18 to 380, 18 to 400, 18 to 420, 18 to 440, 18 to 460, 18 to 480 or 18 to 500 of the sequence according to SEQ ID NO: 1. Preferably, the fragment of human ACE2 consists of amino acids 18 to 520, 18 to 540, 18 to 560, 18 to 580 or 18 to 600 of the sequence according to SEQ ID NO: 1. More preferably, the fragment of human ACE2 consists of amino acids 18 to 605, 18 to 615, 18 to 620, 18 to 640, 18 to 660, 18 to 680 or 18 to 700 of the sequence according to SEQ ID NO: 1. Even more preferably, the fragment of human ACE2 consists of amino acids 18 to 710, 18 to 720 or 18 to 730 of the sequence according to SEQ ID NO: 1.

In one embodiment, the fragment of human ACE2 consists of the amino acid sequence according to SEQ ID NO: 2. The amino acid sequence according to SEQ ID NO: 2 starts at amino acid Q18 and ends with amino acid G732 in the sequence according to SEQ ID NO: 1. The amino acid glycine at the C-terminal end of this fragment provides a high rotational freedom which favours the fusion of the two protein parts and increases the stability of the fusion protein. Additionally, the use of an ACE2 fragment comprising the amino acid sequence which starts at amino acid Q18 and ends with amino acid G732 in the sequence according to SEQ ID NO: 1 provides a better yield than a longer ACE2 fragment.

In one embodiment, the fragment of human ACE2 consists of the complete extracellular domain of human ACE2 which has the amino acid sequence according to SEQ ID NO:3.

In one embodiment, the fragment of human ACE2 consists of the amino acid sequence according to SEQ ID NO: 12. The amino acid sequence according to SEQ ID NO: 12 starts at amino acid Q18 and ends with amino acid G605 in the sequence according to SEQ ID NO: 1.

In one embodiment, the fragment of human ACE2 is N-glycosylated at at least one amino acid residue selected from N53, N90, N103, N322, N432, N546 and N690, the numbering referring to SEQ ID NO: 1. In one embodiment, the fragment of human ACE2 is N-glycosylated at amino acid residues N53, N90 and, N322, the numbering referring to SEQ ID NO: 1. In one embodiment, the fragment of human ACE2 is N-glycosylated at amino acid residues N53, N90, N103, N322, N432, N546 and N690, the numbering referring to SEQ ID NO: 1.

In one embodiment, the fragment of human ACE2 consists of the amino acid sequence according to SEQ ID NO: 2 and is N-glycosylated at at least one amino acid residue selected from N53, N90, N103, N322, N432, N546 and N690, the numbering referring to SEQ ID NO: 1. In one embodiment, the fragment of human ACE2 consists of the amino acid sequence according to SEQ ID NO: 2 and is N-glycosylated at amino acid residues N53, N90 and N322, the numbering referring to SEQ ID NO: 1. In one embodiment, the fragment of human ACE2 consists of the amino acid sequence according to SEQ ID NO: 2 and is N-glycosylated at amino acid residues N53, N90, N103, N322, N432, N546 and N690, the numbering referring to SEQ ID NO: 1.

In one embodiment, the fragment of human ACE2 consists of the amino acid sequence according to SEQ ID NO: 3 and is N-glycosylated at at least one amino acid residue selected from N53, N90, N103, N322, N432, N546 and N690, the numbering referring to SEQ ID NO: 1. In one embodiment, the fragment of human ACE2 consists of the amino acid sequence according to SEQ ID NO: 3 and is N-glycosylated at amino acid residues N53, N90 and N322, the numbering referring to SEQ ID NO: 1. In one embodiment, the fragment of human ACE2 consists of the amino acid sequence according to SEQ ID NO: 3 and is N-glycosylated at amino acid residues N53, N90, N103, N322, N432, N546 and N690, the numbering referring to SEQ ID NO: 1.

The term "N-glycosylated" or "N-glycosylation" means that a glycan structure is attached to the amide nitrogen of an asparagine residue of a protein. A glycan is a branched, flexible chain of carbohydrates and the exact structure of the glycan attached to the asparagine residue of a protein depends on the expression system used for glycoprotein production.

In one embodiment, 70% to 95%, preferably 73% to 92% and most preferably 75% to 90% of the N-glycans on the ACE2 protein have at least one sialic acid molecule attached to the N-glycan, meaning that one terminal galactose molecule is linked to a sialic acid molecule. The sialic acid molecule is attached to the terminal galactose residues of the glycan structure. In one embodiment, 35% to 60% preferably 38% to 55% and most preferably 40% to 50% of the N-glycans on the ACE2 protein have at least two two sialic acid molecules attached to the N-glycan, meaning that two terminal galactose molecules are linked to a sialic acid molecule. In one embodiment, 1% to 10%, preferably 2% to 9%, more preferably 3% to 8% and most preferably 5% to 8% of the N-glycans on the ACE2 protein have at least three sialic acid molecules attached to the N-glycan, meaning that three terminal galactose molecules are linked to a sialic acid molecule. In one embodiment, 0% to 4%, preferably 0.5 % to 3%, more preferably 0.8% to 2.5% and most preferably 1% to 2% of the N-glycans on the ACE2 protein have at least four sialic acid molecules attached to the N-glycans, meaning that four terminal galactose molecules are linked to a sialic acid molecule.

In one embodiment, amino acid residues N53, N90, N103, N322, N432, N546 and N690, the numbering referring to SEQ ID NO: 1, are N-glycosylated and 70% to 95%, preferably 73% to 92% and most preferably 75% to 90% of the N-glycans attached to said amino acid residues have at least one sialic acid molecule attached to the N-glycan, meaning that one terminal galactose molecule is linked to a sialic acid molecule. In one embodiment, amino acid residues N53, N90, N103, N322, N432, N546 and N690, the numbering referring to SEQ ID NO: 1, are N-glycosylated and 35% to 60% preferably 38% to 55% and most preferably 40% to 50% of the N-glycans on the ACE2 protein have at least two two sialic acid molecules attached to the N-glycan, meaning that two terminal galactose molecules are linked to a sialic acid molecule..In one embodiment, amino acid residues N53, N90, N103, N322, N432, N546 and N690, the numbering referring to SEQ ID NO: 1, are N-glycosylated and 1% to 10%, preferably 2% to 9%, more preferably 3% to 8% and most preferably 5% to 8% of the N-glycans on the ACE2 protein have at least three sialic acid molecules attached to the N-glycan, meaning that three terminal galactose molecules are linked to a sialic acid molecule. In one embodiment, amino acid residues N53, N90, N103, N322, N432, N546 and N690, the numbering referring to SEQ ID NO: 1, are N-glycosylated and 0% to 4%, preferably 0.5 % to 3%, more preferably 0.8% to 2.5% and most preferably 1% to 2% of the N-glycans on the ACE2 protein have at least four sialic acid molecules attached to the N-glycans, meaning that four terminal galactose molecules are linked to a sialic acid molecule.

The glycoprotein structure can be analyzed by different methods known to the skilled person, including digestion of the fusion protein and analysis by peptide mapping. Alternatively, hydrophilic interaction liquid chromatography (HILIC) may be performed for glycoprotein analysis.

An increased sialylation leads to an increased half-life of a therapeutic protein (see e.g., Byrne et al. (2007) Drug Discovery Today 12 (7-8): 319-326; Jones et al. (2007) Glycobiology 17(5): 529-540).

The sialylation may be increased by culturing the host cells expressing the fusion protein under specific conditions which increase sialylation. For example, a eukaryotic host cell comprising a recombinant nucleic acid molecule encoding said fusion protein may be cultured in a medium supplemented with one or more of a manganese salt, N-acetylmannosamine or a derivative thereof, galactose, glucosamine or a derivative thereof and DMSO.

It has been shown that the above cell culture additives enhance sialylation of proteins produced in eukaryotic host cells (see, e.g., Liu et al. (2015) World J. Microbiol. Biotechnol. 31(7): 1147-1156; Crowell et al. (2007) Biotechnol. Bioeng. 96(3): 538-549; Lee et al. (2017) Biotechnol. Bioeng. 114(9): 1991-2000); WO 2011/061275; WO 2004/008100).

The sialylation may be increased by culturing the eukaryotic host cell comprising a recombinant nucleic acid molecule encoding said fusion protein in a medium supplemented with 20 to 80 µM of a manganese salt, preferably with 25 to 70 µM of a manganese salt, more preferably with 30 µM to 60 µM of a manganese salt and most preferably with 40 µM of a manganese salt. The sialylation may be increased by culturing the eukaryotic host cell comprising a recombinant nucleic acid molecule encoding said fusion protein in a medium supplemented with 20 to 80 µM of manganese chloride, preferably with 25 to 70 µM of manganese chloride, more preferably with 30 µM to 60 µM of manganese chloride and most preferably with 40 µM of manganese chloride.

The sialylation may be increased by culturing the eukaryotic host cell comprising a recombinant nucleic acid molecule encoding said fusion protein in a medium supplemented with 5 to 30 mM N-acetylmannosamine, preferably with 6 to 25 mM N-acetylmannosamine, more preferably with 8 to 20 mM N-acetylmannosamine and most preferably with 10 mM N-acetylmannosamine.

The sialylation may be increased by culturing the eukaryotic host cell comprising a recombinant nucleic acid molecule encoding said fusion protein in a medium supplemented with 20 to 80 µM of a manganese salt and 5 to 30 mM N-acetylmannosamine, preferably with 25 to 70 µM of a manganese salt and 6 to 25 mM N-acetylmannosamine, more preferably with 30 µM to 60 µM of a manganese salt and 8 to 20 mM N-acetylmannosamine and most preferably with 40 µM of a manganese salt and 10 mM N-acetylmannosamine. The sialylation may be increased by culturing the eukaryotic host cell comprising a recombinant nucleic acid molecule encoding said fusion protein in a medium supplemented with 20 to 80 µM of manganese chloride and 5 to 30 mM N-acetylmannosamine, preferably with 25 to 70 µM of manganese chloride and 6 to 25 mM N-acetylmannosamine, more preferably with 30 µM to 60 µM of manganese chloride and 8 to 20 mM N-acetylmannosamine and most preferably with 40 µM of manganese chloride and 10 mM N-acetylmannosamine.

As used herein, the term "sialylation" refers to the addition of a sialic acid residue to a protein, including a glycoprotein. Sialic acid is a common name for a family of unique nine-carbon monosaccharides, which can be linked to other oligosaccharides. Two family members are N- acetyl neuraminic acid, abbreviated as Neu5Ac or NANA, and N-glycolyl neuraminic acid, abbreviated as Neu5Gc or NGNA. The most common form of sialic acid in humans is NANA.

A "variant" of the fragment of human ACE2 refers to a fragment as defined above, wherein compared to the corresponding sequence in the amino acid sequence of wild-type, full-length human ACE2 according to SEQ ID NO: 1 at least one amino acid residue is different or at least two, at least three, at least four, at least five, at least six, at least seven, at least eight, at least nine, at least ten, at least eleven or at least thirteen amino acids are different. The "variant" of the fragment of human ACE2 comprises one or more amino acid substitutions in the sequence of the fragment of human ACE2. A "variant" of the fragment of human ACE2 does not comprise any amino acid additions or deletions compared to the sequence from which the variant is derived. In one embodiment, the variant the fragment of human ACE2 is a variant of the fragment of human ACE2 according to SEQ ID NO: 2 or 3 and does not comprise any amino acid additions or deletions compared to the sequence according to SEQ ID NO: 2 or 3, i.e. it has the same length as the sequence according to SEQ ID NO: 2 or 3. As used herein, a variant of the fragment of human ACE2 is capable of binding to the S protein of at least one coronavirus, in particular to the S protein of SARS-CoV-2. The binding of a variant of the fragment of human ACE2 to the S protein of at least one coronavirus, in particular to the S protein of SARS-CoV-2, can be determined as described above for fragments of human ACE2.

In one embodiment, the variant of the fragment of human ACE2 differs from the corresponding amino acid sequence in the sequence according to SEQ ID NO: 1 by one amino acid. In another embodiment, the variant of the fragment of human ACE2 differs from the corresponding amino acid sequence in the sequence according to SEQ ID NO: 1 by two amino acids. In still another embodiment, the variant of the fragment of human ACE2 differs from the corresponding amino acid sequence in the sequence according to SEQ ID NO: 1 by three amino acids. In still another embodiment, the variant of the fragment of human ACE2 differs from the corresponding amino acid sequence in the sequence according to SEQ ID NO: 1 by four amino acids. In still another embodiment, the variant of the fragment of human ACE2 differs from the corresponding amino acid sequence in the sequence according to SEQ ID NO: 1 by five amino acids. In still another embodiment, the variant of the fragment of human ACE2 differs from the corresponding amino acid sequence in the sequence according to SEQ ID NO: 1 by six amino acids. In still another embodiment, the variant of the fragment of human ACE2 differs from the corresponding amino acid sequence in the sequence according to SEQ ID NO: 1 by seven amino acids. In still another embodiment, the variant of the fragment of human ACE2 differs from the corresponding amino acid sequence in the sequence according to SEQ ID NO: 1 by eight amino acids. In still another embodiment, the variant of the fragment of human ACE2 differs from the corresponding amino acid sequence in the sequence according to SEQ ID NO: 1 by nine amino acids. In still another embodiment, the variant of the fragment of human ACE2 differs from the corresponding amino acid sequence in the sequence according to SEQ ID NO: 1 by ten amino acids. In still another embodiment, the variant of the fragment of human ACE2 differs from the corresponding amino acid sequence in the sequence according to SEQ ID NO: 1 by eleven amino acids. In still another embodiment, the variant of the fragment of human ACE2 differs from the corresponding amino acid sequence in the sequence according to SEQ ID NO: 1 by twelve amino acids. In still another embodiment, the variant of the fragment of human ACE2 differs from the corresponding amino acid sequence in the sequence according to SEQ ID NO: 1 by thirteen amino acids.

In one embodiment, the variant of the fragment of human ACE2 differs from the corresponding amino acid sequence in the sequence according to SEQ ID NO: 1 by one to thirteen amino acids. In one embodiment, the variant of the fragment of human ACE2 differs from the corresponding amino acid sequence in the sequence according to SEQ ID NO: 1 by one to ten amino acids. In one embodiment, the variant of the fragment of human ACE2 differs from the corresponding amino acid sequence in the sequence according to SEQ ID NO: 1 by one to eight amino acids. In one embodiment, the variant of the fragment of human ACE2 differs from the corresponding amino acid sequence in the sequence according to SEQ ID NO: 1 by one to seven amino acids. In one embodiment, the variant of the fragment of human ACE2 differs from the corresponding amino acid sequence in the sequence according to SEQ ID NO: 1 by one to six amino acids. In one embodiment, the variant of the fragment of human ACE2 differs from the corresponding amino acid sequence in the sequence according to SEQ ID NO: 1 by one to five amino acids. In one embodiment, the variant of the fragment of human ACE2 differs from the corresponding amino acid sequence in the sequence according to SEQ ID NO: 1 by one to four amino acids. In one embodiment, the variant of the fragment of human ACE2 differs from the corresponding amino acid sequence in the sequence according to SEQ ID NO: 1 by one to three amino acids. In one embodiment, the variant of the fragment of human ACE2 differs from the corresponding amino acid sequence in the sequence according to SEQ ID NO: 1 by one or two amino acids.

One variant of the fragment of human ACE2 may be an enzymatically inactive variant. "The enzymatically inactive variant of the fragment of human ACE2" lacks the ability to cleave angiotensin II to Ang1-7. The enzymatic activity of human ACE2 can be determined by methods known to the skilled person. Suitable kits for determining the enzymatic activity of human ACE2 are commercially available, for example from the companies BioVision or Anaspec. By using an enzymatically inactive ACE2 variant any side effects associated with the enzymatic activity of ACE2 such as effects on the cardiovascular system or the regulation of blood pressure are eliminated. Further, the risk of counterbalancing the RAS - MAS equilibrium is reduced.

The enzymatically inactive variant of the fragment of human ACE2 may comprise one or more mutations of amino acids within the catalytic centre of ACE2. In particular, the enzymatically inactive variant of the fragment of human ACE2 comprises a mutation of the wildtype histidine at residue 374 of the sequence according to SEQ ID NO: 1 and/or a mutation of the wildtype histidine at residue 378 of the sequence according to SEQ ID NO: 1. The wild-type histidine may be mutated to any amino acid other than histidine and particularly, the wild-type histidine is mutated to asparagine. Preferably, the enzymatically inactive variant of the fragment of human ACE2 comprises a H374N and a H378N mutation, the numbering referring to the sequence according to SEQ ID NO: 1.

In another embodiment, the enzymatically inactive variant of the fragment of human ACE2 comprises a mutation at one or more of the following amino acid residues, the numbering referring to the sequence according to SEQ ID NO: 1: residue 345 (histidine in wild-type), 273 (arginine in wild-type), 402 (glutamic acid in wild-type) and 505 (histidine in wild-type). In one embodiment, the enzymatically inactive variant of the fragment of human ACE2 comprises a mutation of histidine at residue 345 to alanine or leucine, a mutation of arginine at residue 273 to alanine, glutamine or lysine, a mutation of glutamic acid at residue 402 to alanine and/or a mutation of histidine at residue 505 to alanine or leucine, the numbering referring to the sequence according to SEQ ID NO: 1. In a particular embodiment, the enzymatically inactive variant of the fragment of human ACE2 comprises a mutation of arginine at residue 273 to alanine (also called R273A mutation), the numbering referring to the sequence according to SEQ ID NO: 1. It was found that arginine 273 is critical for substrate binding and that its substitution abolishes enzymatic activity (Guy et al. (2005) FEBS J. 272(14): 3512-3520).

In one embodiment, the fragment of human ACE2 consists of amino acids 18 to 380, 18 to 400, 18 to 420, 18 to 440, 18 to 460, 18 to 480 or 18 to 500 of the sequence according to SEQ ID NO: 1 and comprises a H374N and a H378N mutation, the numbering referring to the sequence according to SEQ ID NO: 1. Preferably, the fragment of human ACE2 consists of amino acids 18 to 520, 18 to 540, 18 to 560, 18 to 580 or 18 to 600 of the sequence according to SEQ ID NO: 1 and comprises a H374N and a H378N mutation, the numbering referring to the sequence according to SEQ ID NO: 1. More preferably, the fragment of human ACE2 consists of amino acids 18 to 615, 18 to 620, 18 to 640, 18 to 660, 18 to 680 or 18 to 700 of the sequence according to SEQ ID NO: 1 and comprises a H374N and a H378N mutation, the numbering referring to the sequence according to SEQ ID NO: 1. Even more preferably, the fragment of human ACE2 consists of amino acids 18 to 710, 18 to 720 or 18 to 730 of the sequence according to SEQ ID NO: 1 and comprises a H374N and a H378N mutation, the numbering referring to the sequence according to SEQ ID NO: 1.

In one embodiment, the fragment of human ACE2 consists of the sequence according to SEQ ID NO: 2 and comprises a H374N and a H378N mutation. In one embodiment, the fragment of human ACE2 consists of the sequence according to SEQ ID NO: 3 and comprises a H374N and a H378N mutation.

In one embodiment, the fragment of human ACE2 consists of amino acids 18 to 380, 18 to 400, 18 to 420, 18 to 440, 18 to 460, 18 to 480 or 18 to 500 of the sequence according to SEQ ID NO: 1 and comprises a R273A mutation, the numbering referring to the sequence according to SEQ ID NO: 1. Preferably, the fragment of human ACE2 consists of amino acids 18 to 520, 18 to 540, 18 to 560, 18 to 580 or 18 to 600 of the sequence according to SEQ ID NO: 1 and comprises a R273A mutation, the numbering referring to the sequence according to SEQ ID NO: 1. More preferably, the fragment of human ACE2 consists of amino acids 18 to 615, 18 to 620, 18 to 640, 18 to 660, 18 to 680 or 18 to 700 of the sequence according to SEQ ID NO: 1 and comprises a R273A mutation, the numbering referring to the sequence according to SEQ ID NO: 1. Even more preferably, the fragment of human ACE2 consists of amino acids 18 to 710, 18 to 720 or 18 to 730 of the sequence according to SEQ ID NO: 1 and comprises a R273A mutation, the numbering referring to the sequence according to SEQ ID NO: 1.

In one embodiment, the fragment of human ACE2 consists of a protein having the amino acid sequence according to SEQ ID NO: 2 and comprises a R273A mutation, the numbering referring to the sequence according to SEQ ID NO: 1. In one embodiment, the fragment of human ACE2 consists of a protein having the amino acid sequence according to SEQ ID NO: 3 and comprises a R273A mutation, the numbering referring to the sequence according to SEQ ID NO: 1.

Another variant of the fragment of human ACE2 may be a variant which inhibits shedding of ACE2. It was shown that ACE2 is shed from human airway epithelia by cleavage of the ACE2 ectodomain and that ADAM17 regulates ACE2 cleavage. Further, a point mutation at leucine 584 of full-length ACE2 which is located in the ectodomain of ACE2 abolished shedding (Jia et al. (2009) Am. J. Physiol. Lung Cell. Mol. Physiol. 297(1): L84-96). Hence, in one embodiment the variant of the fragment of human ACE2 comprises a mutation at leucine 584, the numbering referring to the sequence according to SEQ ID NO: 1. In one embodiment the mutation at leucine 584 is a L584A mutation.

In one embodiment, the fragment of human ACE2 consists of the sequence according to SEQ ID NO: 2 and comprises a L584A mutation, the numbering referring to the sequence according to SEQ ID NO: 1.

In one embodiment, the fragment of human ACE2 consists of the sequence according to SEQ ID NO: 3 and comprises a L584A mutation, the numbering referring to the sequence according to SEQ ID NO: 1.

In one embodiment, the variant of the fragment of human ACE2 comprises a H374N mutation, a H378N mutation and a L584A mutation, the numbering referring to the sequence according to SEQ ID NO: 1.

In one embodiment, the fragment of human ACE2 consists of the sequence according to SEQ ID NO: 2 and comprises a H374N mutation, a H378N mutation and a L584A mutation, the numbering referring to the sequence according to SEQ ID NO: 1.

In one embodiment, the fragment of human ACE2 consists of the sequence according to SEQ ID NO: 3 and comprises a H374N mutation, a H378N mutation and a L584A mutation, the numbering referring to the sequence according to SEQ ID NO: 1.

Another variant of the fragment of human ACE2 may be a variant which inhibits cleavage of ACE2 by the protease TMPRSS2. It was shown that ACE2 proteolysis by TMPRSS2 augments entry of SARS-CoV (Heurich et al. (2014) J. Virol. 88(2): 1293-1307). TMPRSS2 also plays a role in the entry of SARS-CoV-2 into the cells (Hoffmann et al. (2020) Cell 181: 1-10). To abolish cleavage of ACE2 by TMPRSS2, the amino acid residues essential for the cleavage may be mutated. It was shown that arginine and lysine residues within the amino acid region spanning amino acids 697 to 716 of ACE2 are essential for ACE2 cleavage by TMPRSS2 (Heurich et al. (2014) J. Virol. 88(2): 1293-1307). Hence, in one embodiment the variant of the fragment of human ACE2 comprises a mutation at at least one residue selected from amino acids 697, 702, 705, 708, 710 and 716, the numbering referring to SEQ ID NO: 1. Preferably, the variant of the fragment of human ACE2 comprises a mutation at at least two or three residues selected from amino acids 697, 702, 705, 708, 710 and 716, the numbering referring to SEQ ID NO: 1. More preferably, the variant of the fragment of human ACE2 comprises a mutation at at least four or five residues selected from amino acids 697, 702, 705, 708, 710 and 716, the numbering referring to SEQ ID NO: 1. Most preferably, the variant of the fragment of human ACE2 comprises a mutation at residues 697, 702, 705, 708, 710 and 716, the numbering referring to SEQ ID NO: 1. The wild-type amino acid residue at any of these residues may be mutated to any other amino acid and particularly, the wild-type amino acid residue is mutated to alanine.

In one embodiment, the variant of the fragment of human ACE2 comprises at least one of the following mutations: R697A, K702A, R705A, R708A, R710A and R716A, the numbering referring to SEQ ID NO: 1. Preferably, the variant of the fragment of human ACE2 comprises at least two or three of the following mutations: R697A, K702A, R705A, R708A, R710A and R716A, the numbering referring to SEQ ID NO: 1. More preferably, the variant of the fragment of human ACE2 comprises at least four or five of the following mutations: R697A, K702A, R705A, R708A, R710A and R716A, the numbering referring to SEQ ID NO: 1. Most preferably, the variant of the fragment of human ACE2 comprises the following mutations: R697A, K702A, R705A, R708A, R710A and R716A, the numbering referring to SEQ ID NO: 1.

The variant of the fragment of human ACE2 may further comprise mutations at residues 619, 621 and/or 625, the numbering referring to SEQ ID NO: 1. In particular, the variant of the fragment of human ACE2 may further comprise the following mutations: K619A, R621A and/or K625A, the numbering referring to SEQ ID NO: 1.

Hence, in one embodiment, the variant of the fragment of human ACE2 comprises the following mutations: K619A, R621A, K625A, R697A, K702A, R705A, R708A, R710A and R716A, the numbering referring to SEQ ID NO: 1.

In one embodiment, the fragment of human ACE2 consists of the sequence according to SEQ ID NO: 2 and comprises a mutation at at least one residue selected from amino acids 697, 702, 705, 708, 710 and 716, the numbering referring to SEQ ID NO: 1. In one embodiment, the fragment of human ACE2 consists of the sequence according to SEQ ID NO: 2 and comprises a mutation at residues 697, 702, 705, 708, 710 and 716, the numbering referring to SEQ ID NO: 1. In one embodiment, the fragment of human ACE2 consists of the sequence according to SEQ ID NO: 2 and comprises at least one of the following mutations: R697A, K702A, R705A, R708A, R710A and R716A, the numbering referring to SEQ ID NO: 1. In one embodiment, the fragment of human ACE2 consists of the sequence according to SEQ ID NO: 2 and comprises the following mutations: R697A, K702A, R705A, R708A, R710A and R716A, the numbering referring to SEQ ID NO: 1.

In one embodiment, the fragment of human ACE2 consists of the sequence according to SEQ ID NO: 3 and comprises a mutation at at least one residue selected from amino acids 697, 702, 705, 708, 710 and 716, the numbering referring to SEQ ID NO: 1. In one embodiment, the fragment of human ACE2 consists of the sequence according to SEQ ID NO: 3 and comprises a mutation at residues 697, 702, 705, 708, 710 and 716, the numbering referring to SEQ ID NO: 1. In one embodiment, the fragment of human ACE2 consists of the sequence according to SEQ ID NO: 3 and comprises at least one of the following mutations: R697A, K702A, R705A, R708A, R710A and R716A, the numbering referring to SEQ ID NO: 1. In one embodiment, the fragment of human ACE2 consists of the sequence according to SEQ ID NO: 3 and comprises the following mutations: R697A, K702A, R705A, R708A, R710A and R716A, the numbering referring to SEQ ID NO: 1.

In one embodiment, the fragment of human ACE2 consists of the sequence according to SEQ ID NO: 2 and comprises a H374N mutation, a H378N mutation, a L584A mutation and the following mutations: R697A, K702A, R705A, R708A, R710A and R716A, the numbering referring to the sequence according to SEQ ID NO: 1.

In one embodiment, the fragment of human ACE2 consists of the sequence according to SEQ ID NO: 3 and comprises a H374N mutation, a H378N mutation, a L584A mutation and the following mutations: R697A, K702A, R705A, R708A, R710A and R716A, the numbering referring to the sequence according to SEQ ID NO: 1.

In one embodiment, the fragment of human ACE2 consists of the sequence according to SEQ ID NO: 2 and comprises a mutation at at least one residue selected from amino acids 619, 621, 625, 697, 702, 705, 708, 710 and 716, the numbering referring to SEQ ID NO: 1. In one embodiment, the fragment of human ACE2 consists of the sequence according to SEQ ID NO: 2 and comprises a mutation at residues 619, 621, 625, 697, 702, 705, 708, 710 and 716, the numbering referring to SEQ ID NO: 1. In one embodiment, the fragment of human ACE2 consists of the sequence according to SEQ ID NO: 2 and comprises at least one of the following mutations: K619A, R621A, K625A, R697A, K702A, R705A, R708A, R710A and R716A, the numbering referring to SEQ ID NO: 1. In one embodiment, the fragment of human ACE2 consists of the sequence according to SEQ ID NO: 2 and comprises the following mutations: K619A, R621A, K625A, R697A, K702A, R705A, R708A, R710A and R716A, the numbering referring to SEQ ID NO: 1.

In one embodiment, the fragment of human ACE2 consists of the sequence according to SEQ ID NO: 3 and comprises a mutation at at least one residue selected from amino acids 619, 621, 625, 697, 702, 705, 708, 710 and 716, the numbering referring to SEQ ID NO: 1. In one embodiment, the fragment of human ACE2 consists of the sequence according to SEQ ID NO: 3 and comprises a mutation at residues 619, 621, 625, 697, 702, 705, 708, 710 and 716, the numbering referring to SEQ ID NO: 1. In one embodiment, the fragment of human ACE2 consists of the sequence according to SEQ ID NO: 3 and comprises at least one of the following mutations: K619A, R621A, K625A, R697A, K702A, R705A, R708A, R710A and R716A, the numbering referring to SEQ ID NO: 1. In one embodiment, the fragment of human ACE2 consists of the sequence according to SEQ ID NO: 3 and comprises the following mutations: K619A, R621A, K625A, R697A, K702A, R705A, R708A, R710A and R716A, the numbering referring to SEQ ID NO: 1.

In one embodiment, the fragment of human ACE2 consists of the sequence according to SEQ ID NO: 2 and comprises a H374N mutation, a H378N mutation, a L584A mutation and the following mutations: K619A, R621A, K625A, R697A, K702A, R705A, R708A, R710A and R716A, the numbering referring to the sequence according to SEQ ID NO: 1.

In one embodiment, the fragment of human ACE2 consists of the sequence according to SEQ ID NO: 3 and comprises a H374N mutation, a H378N mutation, a L584A mutation and the following mutations: K619A, R621A, K625A, R697A, K702A, R705A, R708A, R710A and R716A, the numbering referring to the sequence according to SEQ ID NO: 1.

Another variant of the fragment of human ACE2 may be a variant which provides an additional cysteine for the formation of disulfide bridges between two ACE2 molecules. The disulfide bridge increases the intrinsic stability of the fusion protein and may also have an effect on the binding of the fusion protein to its target. The additional cysteine may be provided by a substitution of serine 645 in the numbering of SEQ ID NO: 1 with cysteine.

Hence, in one embodiment, the variant of the fragment of human ACE2 comprises a S645C mutation, the numbering referring to SEQ ID NO: 1.

In one embodiment, the fragment of human ACE2 consists of the sequence according to SEQ ID NO: 2 and comprises a S645C mutation, the numbering referring to SEQ ID NO: 1.

In one embodiment, the fragment of human ACE2 consists of the sequence according to SEQ ID NO: 3 and comprises a S645C mutation, the numbering referring to SEQ ID NO: 1. In one embodiment,

In one embodiment, the fragment of human ACE2 consists of a protein having the amino acid sequence according to SEQ ID NO: 2 and comprises a H374N mutation, a H378N mutation, and a S645C mutation, the numbering referring to SEQ ID NO: 1.

In one embodiment, the fragment of human ACE2 consists of a protein having the amino acid sequence according to SEQ ID NO: 3 and comprises a H374N mutation, a H378N mutation, and a S645C mutation, the numbering referring to SEQ ID NO: 1.

In one embodiment, the fragment of human ACE2 consists of a protein having the amino acid sequence according to SEQ ID NO: 2 and comprises a R273A mutation, and a S645C mutation, the numbering referring to SEQ ID NO: 1.

In one embodiment, the fragment of human ACE2 consists of a protein having the amino acid sequence according to SEQ ID NO: 3 and comprises a R273A mutation and a S645C mutation, the numbering referring to SEQ ID NO: 1.

In one embodiment, the fragment of human ACE2 consists of the sequence according to SEQ ID NO: 2 and comprises a H374N mutation, a H378N mutation, a L584A mutation and a S645C mutation, the numbering referring to SEQ ID NO: 1.

In one embodiment, the fragment of human ACE2 consists of the sequence according to SEQ ID NO: 3 and comprises a H374N mutation, a H378N mutation, a L584A mutation and a S645C mutation, the numbering referring to SEQ ID NO: 1.

Another variant of the fragment of human ACE2 may be a variant which inhibits dimerization. Hence, in one embodiment the variant of the fragment of human ACE2 comprises a mutation at amino acid Q139, the numbering referring to SEQ ID NO: 1. In one embodiment the variant of the fragment of human ACE2 comprises a Q139A mutation, the numbering referring to SEQ ID NO: 1.

In one embodiment, the fragment of human ACE2 consists of the sequence according to SEQ ID NO: 2 and comprises a Q139A mutation, the numbering referring to SEQ ID NO: 1.

In one embodiment, the fragment of human ACE2 consists of the sequence according to SEQ ID NO: 3 and comprises a Q139A mutation, the numbering referring to SEQ ID NO: 1.

In one embodiment, the fragment of human ACE2 consists of the sequence according to SEQ ID NO: 2 and comprises a H374N mutation, a H378N mutation, a L584A mutation and a Q139A mutation, the numbering referring to SEQ ID NO: 1.

In one embodiment, the fragment of human ACE2 consists of the sequence according to SEQ ID NO: 3 and comprises a H374N mutation, a H378N mutation, a L584A mutation and a Q139A mutation, the numbering referring to SEQ ID NO: 1.

In one embodiment, the fragment of human ACE2 consists of the sequence according to SEQ ID NO: 12 and comprises a Q139A mutation, the numbering referring to SEQ ID NO: 1.

In one embodiment, the fragment of human ACE2 consists of the sequence according to SEQ ID NO: 12 and comprises a H374N mutation, a H378N mutation, a L584A mutation and a Q139A mutation, the numbering referring to SEQ ID NO: 1.

The second part of the fusion protein comprises the Fc portion of human IgM or a fragment or variant thereof.

In one embodiment, the second part of the fusion protein comprises the Fc portion of human IgM. The full-length Fc portion of human IgM comprises the CH2, CH3, CH4 domains and the tailpiece of human IgM linked together to form the Fc portion. A full-length human IgM antibody additionally comprises the Fab fragment which comprises the heavy chain variable region and the CH1 domain. Preferably, the Fc portion of human IgM used in the fusion protein has the sequence according to SEQ ID NO: 4. The fusion protein forms homodimers containing two polypeptide chains with the same amino acid sequence, preferably the amino acid sequence according to SEQ ID NO: 4, which polypeptide chains are linked by disulfide bonds.

It is known that immunoglobulins of the IgM subclass exist as pentamers and hexamers of homodimers due to disulfide bonds between the Fc domain of the homodimers (Müller et al. (2013) Proc. Natl. Acad. Sci USA 110(25): 10183-10188), resulting in ten or twelve antigen-binding sites that allow them to potently neutralize antigens. Further, immunoglobulins of the IgM subclass do not bind to Fcy receptors. Since it has been discussed that Fcy receptors play a role in antibody-dependent enhancement of viral pathogenesis (see, e.g., Bournazos et al. (2020) Nature Reviews Immunology 20: 633-643), the antibody-dependent enhancement can be avoided by using a fusion protein comprising the Fc portion of human IgM or a variant or fragment of the Fc portion of human IgM.

"A variant of the Fc portion of human IgM" refers to the Fc portion of human IgM which has one or more amino acid substitutions compared to the wild-type Fc portion of human IgM according to SEQ ID NO: 4. In one embodiment, the variant of the Fc portion of human IgM has one to twelve, one to eleven, one to ten, one to nine, one to eight, one to seven, one to six, one to five, one to four, one to three, one or two amino acid substitutions compared to the wild-type Fc portion of human IgM according to SEQ ID NO: 4. In one embodiment, the variant of the Fc portion of human IgM has one, two, three, four, five, six, seven, eight, nine, ten, eleven or twelve amino acid substitutions compared to the wild-type Fc portion of human IgM according to SEQ ID NO: 4. The variant of the Fc region of human IgM is capable of forming multimers, preferably hexamers. The formation of multimers can be determined by size exclusion chromatography coupled to multi-angle light scattering (SEC-MALS) as described in the examples herein.

In one embodiment, the variant of the Fc portion of human IgM comprises a mutation at position 221 and/or 222, the numbering referring to SEQ ID NO: 4. In one embodiment the variant of the fragment of human ACE2 comprises a L221C mutation and/or a H222L mutation, the numbering referring to SEQ ID NO: 4. It has been shown that a cysteine at position 221 is important for the formation of polymeric antibodies. The histidine at position 222 may be mutated to leucine, since a histidine at this position may interfere with disulfide bond formation and thereby with polymeric assembly (Mekhaiel et al. (2011) Scientific Reports 1, Article Number 124).

"A fragment of the Fc portion of human IgM" refers to a polypeptide which lacks one or more amino acids compared to the full-length sequence of the Fc region of human IgM according to SEQ ID NO: 4. The fragment of the Fc region of human IgM is capable of forming multimers, preferably hexamers. The formation of multimers can be determined by size exclusion chromatography coupled to multi-angle light scattering (SEC-MALS) as described in the examples herein.

In one embodiment, the fragment of the Fc portion of human IgM consists of SEQ ID NO: 17. In one embodiment, the fragment of the Fc portion of human IgM consists of SEQ ID NO: 18. In one embodiment, the fragment of the Fc portion of human IgM consists of SEQ ID NO: 19. In one embodiment, the fragment of the Fc portion of human IgM consists of SEQ ID NO: 20 In one embodiment, fragment of the Fc portion of human IgM consists of the sequence according to SEQ ID NO: 7. In one embodiment, the fragment of the Fc portion of human IgM consists of SEQ ID NO: 21. Within the present application, the term "cm4" is used as a synonym for the term "cµ4" and both cm4 and cµ4 refer to the sequence according to SEQ ID NO: 7.

In one embodiment, the first and the are linked by a linker sequence. The linker sequence is a short amino acid sequence which does not have a function on its own and which does not affect the folding of the fusion protein. In one embodiment, the linker sequence comprises five to twenty amino acids, preferably six to 18 amino acids, more preferably seven to 17 amino acids and most preferably eight amino acids.

In one embodiment, the linker sequence consists of small amino acids selected from glycine and serine. An overview of linker sequences is provided in Chen et al. (2013) Adv. Drug Deliv. Rev. 65(10): 1357-1369. In one embodiment, the linker sequence comprises five to twenty amino acids and consists of small amino acids selected from glycine and serine. In one embodiment, the linker sequence comprises six to 18 amino acids and consists of small amino acids selected from glycine and serine. In one embodiment, the linker sequence comprises seven to 17 amino acids and consists of small amino acids selected from glycine and serine. In one embodiment, the linker sequence comprises eight amino acids and consists of small amino acids selected from glycine and serine. In one embodiment, the linker sequence has the amino acid sequence according to SEQ ID NO: 5.

In another embodiment, the linker sequence is the hinge region of a human IgG antibody. In one embodiment, the linker sequence is the hinge region of a human IgG1 antibody, a human IgG4 antibody or a human IgG2 antibody. In one embodiment, the hinge region is the hinge region of a human IgG4 antibody and has the amino acid sequence according to SEQ ID NO: 14. In one embodiment, the hinge region is the hinge region of a human IgG1 antibody and has the amino acid sequence according to SEQ ID NO: 15. In one embodiment, the hinge region is the hinge region of a human IgG2 antibody and has the amino acid sequence according to SEQ ID NO: 16. An overview of antibody structure and the role of the hinge region is provided in the Chapter "Antibody structure-function relationships" in Therapeutic Antibody Engineering - Current and Future Advances Driving the Strongest Growth Area in the Pharmaceutical Industry, Woodhead Publishing Series in Biomedicine 2012, pages 37-56, 459-595 (available at Antibody structure-function relationships - ScienceDirect).

In one embodiment, the fusion protein comprises a signal peptide which is located N-terminal of the ACE2 part of the fusion protein. The signal peptide functions to target the protein to the endoplasmic reticulum and ultimately to secretion from the cell. The skilled person knows suitable signal peptides. In one embodiment, the signal peptide is selected from a human albumin signal peptide, a human chymotrypsinogen signal peptide, a human interleukin-2 signal peptide, a human trypsinogen-2 signal peptide or a heavy or light chain signal peptide. In a preferred embodiment, the signal peptide is a human albumin signal peptide. In a particularly preferred embodiment, the signal peptide is a human albumin signal peptide according to SEQ ID NO: 6.

In a particular embodiment, the fusion protein has the amino acid sequence according to SEQ ID NO: 8 which comprises the signal peptide according to SEQ ID NO: 6, amino acids 18 to 740 of human ACE2 (SEQ ID NO: 3), the linker sequence according to SEQ ID NO: 5 and the Fc portion of human IgM according to SEQ ID NO: 4.

In a particular embodiment, the fusion protein of comprises amino acids 18 to 740 of human ACE2 (SEQ ID NO: 3), the linker sequence according to SEQ ID NO: 5 and the Fc portion of human IgM according to SEQ ID NO: 4.

In a particular embodiment, the fusion protein comprises amino acids 18 to 740 of human ACE2 (SEQ ID NO: 3), a linker sequence and the Fc portion of human IgM according to SEQ ID NO: 4.

In another particular embodiment, the fusion protein has the amino acid sequence according to SEQ ID NO: 9 which comprises the signal peptide according to SEQ ID NO: 6, amino acids 18 to 740 of human ACE2 (SEQ ID NO: 3), the linker sequence according to SEQ ID NO: 5 and the fragment of the Fc portion of human IgM according to SEQ ID NO: 7.

In another particular embodiment, the fusion protein comprises amino acids 18 to 740 of human ACE2 (SEQ ID NO: 3), the linker sequence according to SEQ ID NO: 5 and the fragment of the Fc portion of human IgM according to SEQ ID NO: 7.

In another particular embodiment, the fusion protein comprises amino acids 18 to 740 of human ACE2 (SEQ ID NO: 3), a linker sequence and the fragment of the Fc portion of human IgM according to SEQ ID NO: 7.

In a particular embodiment, the liquid pharmaceutical composition of the present invention comprises multimers of the fusion protein of a fragment of human ACE2 and the Fc portion of human IgM or a fragment or variant of the Fc portion of human IgM. The term "multimer" is intended to mean a structure wherein two to twelve fusion proteins of the present invention are associated by disulfide bonds. In one particular embodiment, the term multimer means hexamer, i.e. six fusion proteins of the present invention are associated by disulfide bonds. It was surprisingly shown that the IgM fusion proteins are able to predominantly form hexamers. By the formation of hexamers the antigen binding can be enhanced.

In one embodiment, the present invention also relates to a composition comprising predominantly the hexameric form of the fusion protein of a fragment of human ACE2 and the Fc portion of human IgM or a fragment of the Fc portion of human IgM, i.e. at least 51% or 55%, preferably at least 60% or 65%, more preferably at least 70% or 75%% and most preferably 80%, 85% or 90% of the fusion protein of a fragment of human ACE2 and the Fc portion of human IgM or a fragment of the Fc portion of human IgM in the composition is present as a hexamer. Methods for separating the hexamer made of six homodimers from the free homodimers and for distinguishing between homodimers and hexamer of homodimers include affinity chromatography and size exclusion chromatography.

In one embodiment, the buffer is an acetate buffer and the fusion protein has the amino acid sequence according to SEQ ID NO: 8.

In one embodiment, the buffer is an acetate buffer having a pH within the range of 5.2 to 6.0 and the fusion protein has the amino acid sequence according to SEQ ID NO: 8.

In one embodiment, the buffer is an acetate buffer with a pH of 5.4 and the fusion protein has the amino acid sequence according to SEQ ID NO: 8.

In one embodiment, the buffer is an acetate buffer with a pH of 5.8 and the fusion protein has the amino acid sequence according to SEQ ID NO: 8.

In one embodiment, the buffer is an acetate buffer with a concentration of 10 to 20 mM and the fusion protein has the amino acid sequence according to SEQ ID NO: 8.

In one embodiment, the buffer is an acetate buffer with a concentration of 10 to 20 mM and a pH within the range of 5.2 to 6.0 and the fusion protein has the amino acid sequence according to SEQ ID NO: 8.

In one embodiment, the buffer is an acetate buffer with a concentration of 10 to 20 mM and a pH of 5.4 and the fusion protein has the amino acid sequence according to SEQ ID NO: 8.

In one embodiment, the buffer is an acetate buffer with a concentration of 10 to 20 mM and a pH of 5.8 and the fusion protein has the amino acid sequence according to SEQ ID NO: 8.

In one embodiment, the buffer is an acetate buffer with a concentration of 10 mM and a pH of 5.8, the fusion protein has the amino acid sequence according to SEQ ID NO: 8 and the sugar is trehalose with a concentration of 250 mM.

In one embodiment, the buffer is an acetate buffer with a concentration of 10 mM and a pH of 5.4, the fusion protein has the amino acid sequence according to SEQ ID NO: 8 and the sugar is trehalose with a concentration of 250 mM.

In one embodiment, the buffer is an acetate buffer with a concentration of 10 mM and a pH of 5.4, the fusion protein has the amino acid sequence according to SEQ ID NO: 8 and the surfactant is polysorbate 20 with a concentration of 0.02% (w/v).

In one embodiment, the buffer is an acetate buffer with a concentration of 10 mM and a pH of 5.8, the fusion protein has the amino acid sequence according to SEQ ID NO: 8 and the surfactant is polysorbate 20 with a concentration of 0.02% (w/v).

In one embodiment, the buffer is an acetate buffer with a concentration of 10 mM and a pH of 5.4, the fusion protein has the amino acid sequence according to SEQ ID NO: 8, the sugar is trehalose with a concentration of 250 mM and the surfactant is polysorbate 20 with a concentration of 0.02% (w/v).

In one embodiment, the buffer is an acetate buffer with a concentration of 10 mM and a pH of 5.8, the fusion protein has the amino acid sequence according to SEQ ID NO: 8, the sugar is trehalose with a concentration of 250 mM and the surfactant is polysorbate 20 with a concentration of 0.02% (w/v).

In one embodiment, the buffer is an acetate buffer with a concentration of 10 mM and a pH of 5.4, the fusion protein has the amino acid sequence according to SEQ ID NO: 8, the sugar is trehalose with a concentration of 250 mM, the surfactant is polysorbate 20 with a concentration of 0.02% (w/v) and the liquid pharmaceutical composition further comprises L-arginine.

In one embodiment, the buffer is an acetate buffer with a concentration of 10 mM and a pH of 5.8, the fusion protein has the amino acid sequence according to SEQ ID NO: 8, the sugar is trehalose with a concentration of 250 mM and the surfactant is polysorbate 20 with a concentration of 0.02% (w/v) and the liquid pharmaceutical composition further comprises L-arginine.

In one embodiment, the buffer is an acetate buffer with a concentration of 10 mM and a pH of 5.4, the fusion protein has the amino acid sequence according to SEQ ID NO: 8, the sugar is trehalose with a concentration of 250 mM, the surfactant is polysorbate 20 with a concentration of 0.02% (w/v) and the liquid pharmaceutical composition further comprises 30 mM L-arginine.

In one embodiment, the buffer is an acetate buffer with a concentration of 10 mM and a pH of 5.8, the fusion protein has the amino acid sequence according to SEQ ID NO: 8, the sugar is trehalose with a concentration of 250 mM and the surfactant is polysorbate 20 with a concentration of 0.02% (w/v) and the liquid pharmaceutical composition further comprises 30 mM L-arginine.

In one embodiment, the buffer is an acetate buffer with a concentration of 10 mM and a pH of 5.4, the fusion protein has the amino acid sequence according to SEQ ID NO: 8, the sugar is trehalose with a concentration of 250 mM, the surfactant is polysorbate 20 with a concentration of 0.02% (w/v) and the liquid pharmaceutical composition further comprises sodium chloride.

In one embodiment, the buffer is an acetate buffer with a concentration of 10 mM and a pH of 5.8, the fusion protein has the amino acid sequence according to SEQ ID NO: 8, the sugar is trehalose with a concentration of 250 mM and the surfactant is polysorbate 20 with a concentration of 0.02% (w/v) and the liquid pharmaceutical composition further comprises sodium chloride.

In one embodiment, the buffer is an acetate buffer with a concentration of 10 mM and a pH of 5.4, the fusion protein has the amino acid sequence according to SEQ ID NO: 8, the sugar is trehalose with a concentration of 250 mM, the surfactant is polysorbate 20 with a concentration of 0.02% (w/v) and the liquid pharmaceutical composition further comprises 50 mM sodium chloride.

In one embodiment, the buffer is an acetate buffer with a concentration of 10 mM and a pH of 5.8, the fusion protein has the amino acid sequence according to SEQ ID NO: 8, the sugar is trehalose with a concentration of 250 mM and the surfactant is polysorbate 20 with a concentration of 0.02% (w/v) and the liquid pharmaceutical composition further comprises 50 mM sodium chloride.

In one embodiment, the buffer is an acetate buffer with a concentration of 10 mM and a pH of 5.4, the fusion protein has the amino acid sequence according to SEQ ID NO: 8, the sugar is trehalose with a concentration of 250 mM, the surfactant is polysorbate 20 with a concentration of 0.02% (w/v) and the liquid pharmaceutical composition further comprises sodium chloride and L-arginine.

In one embodiment, the buffer is an acetate buffer with a concentration of 10 mM and a pH of 5.8, the fusion protein has the amino acid sequence according to SEQ ID NO: 8, the sugar is trehalose with a concentration of 250 mM and the surfactant is polysorbate 20 with a concentration of 0.02% (w/v) and the liquid pharmaceutical composition further comprises sodium chloride and L-arginine.

In one embodiment, the buffer is an acetate buffer with a concentration of 10 mM and a pH of 5.4, the fusion protein has the amino acid sequence according to SEQ ID NO: 8, the sugar is trehalose with a concentration of 250 mM, the surfactant is polysorbate 20 with a concentration of 0.02% (w/v) and the liquid pharmaceutical composition further comprises 50 mM sodium chloride and 30 mM L-arginine.

In one embodiment, the buffer is an acetate buffer with a concentration of 10 mM and a pH of 5.8, the fusion protein has the amino acid sequence according to SEQ ID NO: 8, the sugar is trehalose with a concentration of 250 mM and the surfactant is polysorbate 20 with a concentration of 0.02% (w/v) and the liquid pharmaceutical composition further comprises 50 mM sodium chloride and 30 mM L-arginine.

In one embodiment, the buffer is an acetate buffer with a concentration of 10 mM and a pH of 5.4, the fusion protein has the amino acid sequence according to SEQ ID NO: 8, the sugar is trehalose with a concentration of 250 mM, the surfactant is polysorbate 20 with a concentration of 0.02% (w/v) and the liquid pharmaceutical composition further comprises L-methionine.

In one embodiment, the buffer is an acetate buffer with a concentration of 10 mM and a pH of 5.8, the fusion protein has the amino acid sequence according to SEQ ID NO: 8, the sugar is trehalose with a concentration of 250 mM and the surfactant is polysorbate 20 with a concentration of 0.02% (w/v) and the liquid pharmaceutical composition further comprises L-methionine.

In one embodiment, the buffer is an acetate buffer with a concentration of 10 mM and a pH of 5.4, the fusion protein has the amino acid sequence according to SEQ ID NO: 8, the sugar is trehalose with a concentration of 250 mM, the surfactant is polysorbate 20 with a concentration of 0.02% (w/v) and the liquid pharmaceutical composition further comprises 5 mM L-methionine.

In one embodiment, the buffer is an acetate buffer with a concentration of 10 mM and a pH of 5.8, the fusion protein has the amino acid sequence according to SEQ ID NO: 8, the sugar is trehalose with a concentration of 250 mM and the surfactant is polysorbate 20 with a concentration of 0.02% (w/v) and the liquid pharmaceutical composition further comprises 5 mM L-methionine.

In one embodiment, the buffer is an acetate buffer with a concentration of 10 mM and a pH of 5.4, the fusion protein has the amino acid sequence according to SEQ ID NO: 8, the sugar is trehalose with a concentration of 250 mM, the surfactant is polysorbate 20 with a concentration of 0.02% (w/v) and the liquid pharmaceutical composition further comprises sodium chloride, L-methionine and L-arginine.

In one embodiment, the buffer is an acetate buffer with a concentration of 10 mM and a pH of 5.8, the fusion protein has the amino acid sequence according to SEQ ID NO: 8, the sugar is trehalose with a concentration of 250 mM and the surfactant is polysorbate 20 with a concentration of 0.02% (w/v) and the liquid pharmaceutical composition further comprises sodium chloride, L-methionine and L-arginine.

In one embodiment, the buffer is an acetate buffer with a concentration of 10 mM and a pH of 5.4, the fusion protein has the amino acid sequence according to SEQ ID NO: 8, the sugar is trehalose with a concentration of 250 mM, the surfactant is polysorbate 20 with a concentration of 0.02% (w/v) and the liquid pharmaceutical composition further comprises 50 mM sodium chloride, 5 mM L-methionine and 30 mM L-arginine.

In one embodiment, the buffer is an acetate buffer with a concentration of 10 mM and a pH of 5.8, the fusion protein has the amino acid sequence according to SEQ ID NO: 8, the sugar is trehalose with a concentration of 250 mM and the surfactant is polysorbate 20 with a concentration of 0.02% (w/v) and the liquid pharmaceutical composition further comprises 50 mM sodium chloride, 5 mM L-methionine and 30 mM L-arginine.

In one embodiment, the buffer is an acetate buffer and the fusion protein has the amino acid sequence according to SEQ ID NO: 9.

In one embodiment, the buffer is an acetate buffer having a pH within the range of 5.2 to 6.0 and the fusion protein has the amino acid sequence according to SEQ ID NO: 9.

In one embodiment, the buffer is an acetate buffer with a pH of 5.4 and the fusion protein has the amino acid sequence according to SEQ ID NO: 9.

In one embodiment, the buffer is an acetate buffer with a pH of 5.8 and the fusion protein has the amino acid sequence according to SEQ ID NO: 9.

In one embodiment, the buffer is an acetate buffer with a concentration of 10 to 20 mM and the fusion protein has the amino acid sequence according to SEQ ID NO: 9.

In one embodiment, the buffer is an acetate buffer with a concentration of 10 to 20 mM and a pH within the range of 5.2 to 6.0 and the fusion protein has the amino acid sequence according to SEQ ID NO: 9.

In one embodiment, the buffer is an acetate buffer with a concentration of 10 to 20 mM and a pH of 5.4 and the fusion protein has the amino acid sequence according to SEQ ID NO: 9.

In one embodiment, the buffer is an acetate buffer with a concentration of 10 mM and a pH of 5.4 and the fusion protein has the amino acid sequence according to SEQ ID NO: 9.

In one embodiment, the buffer is an acetate buffer with a concentration of 10 to 20 mM and a pH of 5.8 and the fusion protein has the amino acid sequence according to SEQ ID NO: 9.

In one embodiment, the buffer is an acetate buffer with a concentration of 10 mM and a pH of 5.8 and the fusion protein has the amino acid sequence according to SEQ ID NO: 9.

In one embodiment, the buffer is an acetate buffer with a concentration of 10 mM and a pH of 5.4, the fusion protein has the amino acid sequence according to SEQ ID NO: 9 and the sugar is trehalose with a concentration of 250 mM.

In one embodiment, the buffer is an acetate buffer with a concentration of 10 mM and a pH of 5.4, the fusion protein has the amino acid sequence according to SEQ ID NO: 9 and the surfactant is polysorbate 20 with a concentration of 0.02% (w/v).

In one embodiment, the buffer is an acetate buffer with a concentration of 10 mM and a pH of 5.4, the fusion protein has the amino acid sequence according to SEQ ID NO: 9, the sugar is trehalose with a concentration of 250 mM and the surfactant is polysorbate 20 with a concentration of 0.02% (w/v).

In one embodiment, the buffer is an acetate buffer with a concentration of 10 mM and a pH of 5.8, the fusion protein has the amino acid sequence according to SEQ ID NO: 9 and the sugar is trehalose with a concentration of 250 mM.

In one embodiment, the buffer is an acetate buffer with a concentration of 10 mM and a pH of 5.8, the fusion protein has the amino acid sequence according to SEQ ID NO: 9 and the surfactant is polysorbate 20 with a concentration of 0.02% (w/v).

In one embodiment, the buffer is an acetate buffer with a concentration of 10 mM and a pH of 5.8, the fusion protein has the amino acid sequence according to SEQ ID NO: 9, the sugar is trehalose with a concentration of 250 mM and the surfactant is polysorbate 20 with a concentration of 0.02% (w/v).

In one embodiment, the buffer is an acetate buffer with a concentration of 10 mM and a pH of 5.4, the fusion protein has the amino acid sequence according to SEQ ID NO: 9, the sugar is trehalose with a concentration of 250 mM, the surfactant is polysorbate 20 with a concentration of 0.02% (w/v) and the liquid pharmaceutical composition further comprises L-arginine.

In one embodiment, the buffer is an acetate buffer with a concentration of 10 mM and a pH of 5.8, the fusion protein has the amino acid sequence according to SEQ ID NO: 9, the sugar is trehalose with a concentration of 250 mM and the surfactant is polysorbate 20 with a concentration of 0.02% (w/v) and the liquid pharmaceutical composition further comprises L-arginine.

In one embodiment, the buffer is an acetate buffer with a concentration of 10 mM and a pH of 5.4, the fusion protein has the amino acid sequence according to SEQ ID NO: 9, the sugar is trehalose with a concentration of 250 mM, the surfactant is polysorbate 20 with a concentration of 0.02% (w/v) and the liquid pharmaceutical composition further comprises 30 mM L-arginine.

In one embodiment, the buffer is an acetate buffer with a concentration of 10 mM and a pH of 5.8, the fusion protein has the amino acid sequence according to SEQ ID NO: 9, the sugar is trehalose with a concentration of 250 mM and the surfactant is polysorbate 20 with a concentration of 0.02% (w/v) and the liquid pharmaceutical composition further comprises 30 mM L-arginine.

In one embodiment, the buffer is an acetate buffer with a concentration of 10 mM and a pH of 5.4, the fusion protein has the amino acid sequence according to SEQ ID NO: 9, the sugar is trehalose with a concentration of 250 mM, the surfactant is polysorbate 20 with a concentration of 0.02% (w/v) and the liquid pharmaceutical composition further comprises sodium chloride.

In one embodiment, the buffer is an acetate buffer with a concentration of 10 mM and a pH of 5.8, the fusion protein has the amino acid sequence according to SEQ ID NO: 9, the sugar is trehalose with a concentration of 250 mM and the surfactant is polysorbate 20 with a concentration of 0.02% (w/v) and the liquid pharmaceutical composition further comprises sodium chloride.

In one embodiment, the buffer is an acetate buffer with a concentration of 10 mM and a pH of 5.4, the fusion protein has the amino acid sequence according to SEQ ID NO: 9, the sugar is trehalose with a concentration of 250 mM, the surfactant is polysorbate 20 with a concentration of 0.02% (w/v) and the liquid pharmaceutical composition further comprises 50 mM sodium chloride.

In one embodiment, the buffer is an acetate buffer with a concentration of 10 mM and a pH of 5.8, the fusion protein has the amino acid sequence according to SEQ ID NO: 9, the sugar is trehalose with a concentration of 250 mM and the surfactant is polysorbate 20 with a concentration of 0.02% (w/v) and the liquid pharmaceutical composition further comprises 50 mM sodium chloride.

In one embodiment, the buffer is an acetate buffer with a concentration of 10 mM and a pH of 5.4, the fusion protein has the amino acid sequence according to SEQ ID NO: 9, the sugar is trehalose with a concentration of 250 mM, the surfactant is polysorbate 20 with a concentration of 0.02% (w/v) and the liquid pharmaceutical composition further comprises sodium chloride and L-arginine.

In one embodiment, the buffer is an acetate buffer with a concentration of 10 mM and a pH of 5.8, the fusion protein has the amino acid sequence according to SEQ ID NO: 9, the sugar is trehalose with a concentration of 250 mM and the surfactant is polysorbate 20 with a concentration of 0.02% (w/v) and the liquid pharmaceutical composition further comprises sodium chloride and L-arginine.

In one embodiment, the buffer is an acetate buffer with a concentration of 10 mM and a pH of 5.4, the fusion protein has the amino acid sequence according to SEQ ID NO: 9, the sugar is trehalose with a concentration of 250 mM, the surfactant is polysorbate 20 with a concentration of 0.02% (w/v) and the liquid pharmaceutical composition further comprises 50 mM sodium chloride and 30 mM L-arginine.

In one embodiment, the buffer is an acetate buffer with a concentration of 10 mM and a pH of 5.8, the fusion protein has the amino acid sequence according to SEQ ID NO: 9, the sugar is trehalose with a concentration of 250 mM and the surfactant is polysorbate 20 with a concentration of 0.02% (w/v) and the liquid pharmaceutical composition further comprises 50 mM sodium chloride and 30 mM L-arginine.

In one embodiment, the buffer is an acetate buffer with a concentration of 10 mM and a pH of 5.4, the fusion protein has the amino acid sequence according to SEQ ID NO: 9, the sugar is trehalose with a concentration of 250 mM, the surfactant is polysorbate 20 with a concentration of 0.02% (w/v) and the liquid pharmaceutical composition further comprises L-methionine.

In one embodiment, the buffer is an acetate buffer with a concentration of 10 mM and a pH of 5.8, the fusion protein has the amino acid sequence according to SEQ ID NO: 9, the sugar is trehalose with a concentration of 250 mM and the surfactant is polysorbate 20 with a concentration of 0.02% (w/v) and the liquid pharmaceutical composition further comprises L-methionine.

In one embodiment, the buffer is an acetate buffer with a concentration of 10 mM and a pH of 5.4, the fusion protein has the amino acid sequence according to SEQ ID NO: 9, the sugar is trehalose with a concentration of 250 mM, the surfactant is polysorbate 20 with a concentration of 0.02% (w/v) and the liquid pharmaceutical composition further comprises 5 mM L-methionine.

In one embodiment, the buffer is an acetate buffer with a concentration of 10 mM and a pH of 5.8, the fusion protein has the amino acid sequence according to SEQ ID NO: 9, the sugar is trehalose with a concentration of 250 mM and the surfactant is polysorbate 20 with a concentration of 0.02% (w/v) and the liquid pharmaceutical composition further comprises 5 mM L-methionine.

In one embodiment, the buffer is an acetate buffer with a concentration of 10 mM and a pH of 5.4, the fusion protein has the amino acid sequence according to SEQ ID NO: 9, the sugar is trehalose with a concentration of 250 mM, the surfactant is polysorbate 20 with a concentration of 0.02% (w/v) and the liquid pharmaceutical composition further comprises sodium chloride, L-methionine and L-arginine.

In one embodiment, the buffer is an acetate buffer with a concentration of 10 mM and a pH of 5.8, the fusion protein has the amino acid sequence according to SEQ ID NO: 9, the sugar is trehalose with a concentration of 250 mM and the surfactant is polysorbate 20 with a concentration of 0.02% (w/v) and the liquid pharmaceutical composition further comprises sodium chloride, L-methionine and L-arginine.

In one embodiment, the buffer is an acetate buffer with a concentration of 10 mM and a pH of 5.4, the fusion protein has the amino acid sequence according to SEQ ID NO: 9, the sugar is trehalose with a concentration of 250 mM, the surfactant is polysorbate 20 with a concentration of 0.02% (w/v) and the liquid pharmaceutical composition further comprises 50 mM sodium chloride, 5 mM L-methionine and 30 mM L-arginine.

In one embodiment, the buffer is an acetate buffer with a concentration of 10 mM and a pH of 5.8, the fusion protein has the amino acid sequence according to SEQ ID NO: 9, the sugar is trehalose with a concentration of 250 mM and the surfactant is polysorbate 20 with a concentration of 0.02% (w/v) and the liquid pharmaceutical composition further comprises 50 mM sodium chloride, 5 mM L-methionine and 30 mM L-arginine.

In one embodiment, the buffer is an acetate buffer and the fusion protein has the amino acid sequence according to SEQ ID NO: 8, wherein the ACE2 part of the fusion protein is N-glycosylated and 70% to 95% of the N-glycans on the ACE2 part have at least one sialic acid molecule attached thereto.

In one embodiment, the buffer is an acetate buffer having a pH within the range of 5.6 to 6.0 and the fusion protein has the amino acid sequence according to SEQ ID NO: 8, wherein the ACE2 part of the fusion protein is N-glycosylated and 70% to 95% of the N-glycans on the ACE2 part have at least one sialic acid molecule attached thereto.

In one embodiment, the buffer is an acetate buffer with a pH of 5.8 and the fusion protein has the amino acid sequence according to SEQ ID NO: 8, wherein the ACE2 part of the fusion protein is N-glycosylated and 70% to 95% of the N-glycans on the ACE2 part have at least one sialic acid molecule attached thereto.

In one embodiment, the buffer is an acetate buffer with a concentration of 10 to 20 mM and the fusion protein has the amino acid sequence according to SEQ ID NO: 8, wherein the ACE2 part of the fusion protein is N-glycosylated and 70% to 95% of the N-glycans on the ACE2 part have at least one sialic acid molecule attached thereto.

In one embodiment, the buffer is an acetate buffer with a concentration of 10 to 20 mM and a pH within the range of 5.6 to 6.0 and the fusion protein has the amino acid sequence according to SEQ ID NO: 8, wherein the ACE2 part of the fusion protein is N-glycosylated and 70% to 95% of the N-glycans on the ACE2 part have at least one sialic acid molecule attached thereto.

In one embodiment, the buffer is an acetate buffer with a concentration of 10 to 20 mM and a pH of 5.8 and the fusion protein has the amino acid sequence according to SEQ ID NO: 8, wherein the ACE2 part of the fusion protein is N-glycosylated and 70% to 95% of the N-glycans on the ACE2 part have at least one sialic acid molecule attached thereto.

In one embodiment, the buffer is an acetate buffer with a concentration of 10 mM and a pH of 5.8 and the fusion protein has the amino acid sequence according to SEQ ID NO: 8, wherein the ACE2 part of the fusion protein is N-glycosylated and 70% to 95% of the N-glycans on the ACE2 part have at least one sialic acid molecule attached thereto.

In one embodiment, the buffer is an acetate buffer with a concentration of 10 mM and a pH of 5.8, the fusion protein has the amino acid sequence according to SEQ ID NO: 8, wherein the ACE2 part of the fusion protein is N-glycosylated and 70% to 95% of the N-glycans on the ACE2 part have at least one sialic acid molecule attached thereto, and the sugar is trehalose with a concentration of 250 mM.

In one embodiment, the buffer is an acetate buffer with a concentration of 10 mM and a pH of 5.8, the fusion protein has the amino acid sequence according to SEQ ID NO: 8, wherein the ACE2 part of the fusion protein is N-glycosylated and 70% to 95% of the N-glycans on the ACE2 part have at least one sialic acid molecule attached thereto, and the surfactant is polysorbate 20 with a concentration of 0.02% (w/v).

In one embodiment, the buffer is an acetate buffer with a concentration of 10 mM and a pH of 5.8, the fusion protein has the amino acid sequence according to SEQ ID NO: 8, wherein the ACE2 part of the fusion protein is N-glycosylated and 70% to 95% of the N-glycans on the ACE2 part have at least one sialic acid molecule attached thereto, the sugar is trehalose with a concentration of 250 mM and the surfactant is polysorbate 20 with a concentration of 0.02% (w/v).

In one embodiment, the buffer is an acetate buffer and the fusion protein has the amino acid sequence according to SEQ ID NO: 9, wherein the ACE2 part of the fusion protein is N-glycosylated and 70% to 95% of the N-glycans on the ACE2 part have at least one sialic acid molecule attached thereto.

In one embodiment, the buffer is an acetate buffer having a pH within the range of 5.6 to 6.0 and the fusion protein has the amino acid sequence according to SEQ ID NO: 9, wherein the ACE2 part of the fusion protein is N-glycosylated and 70% to 95% of the N-glycans on the ACE2 part have at least one sialic acid molecule attached thereto.

In one embodiment, the buffer is an acetate buffer with a pH of 5.8 and the fusion protein has the amino acid sequence according to SEQ ID NO: 9, wherein the ACE2 part of the fusion protein is N-glycosylated and 70% to 95% of the N-glycans on the ACE2 part have at least one sialic acid molecule attached thereto.

In one embodiment, the buffer is an acetate buffer with a concentration of 10 to 20 mM and the fusion protein has the amino acid sequence according to SEQ ID NO: 9, wherein the ACE2 part of the fusion protein is N-glycosylated and 70% to 95% of the N-glycans on the ACE2 part have at least one sialic acid molecule attached thereto.

In one embodiment, the buffer is an acetate buffer with a concentration of 10 to 20 mM and a pH within the range of 5.6 to 6.0 and the fusion protein has the amino acid sequence according to SEQ ID NO: 9, wherein the ACE2 part of the fusion protein is N-glycosylated and 70% to 95% of the N-glycans on the ACE2 part have at least one sialic acid molecule attached thereto.

In one embodiment, the buffer is an acetate buffer with a concentration of 10 to 20 mM and a pH of 5.8 and the fusion protein has the amino acid sequence according to SEQ ID NO: 9, wherein the ACE2 part of the fusion protein is N-glycosylated and 70% to 95% of the N-glycans on the ACE2 part have at least one sialic acid molecule attached thereto.

In one embodiment, the buffer is an acetate buffer with a concentration of 10 mM and a pH of 5.8 and the fusion protein has the amino acid sequence according to SEQ ID NO: 9, wherein the ACE2 part of the fusion protein is N-glycosylated and 70% to 95% of the N-glycans on the ACE2 part have at least one sialic acid molecule attached thereto.

In one embodiment, the buffer is an acetate buffer with a concentration of 10 mM and a pH of 5.8, the fusion protein has the amino acid sequence according to SEQ ID NO: 9, wherein the ACE2 part of the fusion protein is N-glycosylated and 70% to 95% of the N-glycans on the ACE2 part have at least one sialic acid molecule attached thereto, and the sugar is trehalose with a concentration of 250 mM.

In one embodiment, the buffer is an acetate buffer with a concentration of 10 mM and a pH of 5.8, the fusion protein has the amino acid sequence according to SEQ ID NO: 9, wherein the ACE2 part of the fusion protein is N-glycosylated and 70% to 95% of the N-glycans on the ACE2 part have at least one sialic acid molecule attached thereto, and the surfactant is polysorbate 20 with a concentration of 0.02% (w/v).

In one embodiment, the buffer is an acetate buffer with a concentration of 10 mM and a pH of 5.8, the fusion protein has the amino acid sequence according to SEQ ID NO: 9, wherein the ACE2 part of the fusion protein is N-glycosylated and 70% to 95% of the N-glycans on the ACE2 part have at least one sialic acid molecule attached thereto, the sugar is trehalose with a concentration of 250 mM and the surfactant is polysorbate 20 with a concentration of 0.02% (w/v).

The concentration of the ACE2 Fc fusion protein in the liquid pharmaceutical compositions of the present invention is 1-60 mg/mL, preferably 5-50 mg/mL or 8-40 mg/mL, more preferably 10-30 mg/mL or 15-25 mg/mL, and most preferably 20 mg/mL. In another embodiment the concentration of the ACE2 Fc fusion protein in the liquid pharmaceutical compositions of the present invention is 20-60 mg/mL, preferably 30-50 mg/mL, more preferably 40 mg/mL.

The liquid pharmaceutical composition of the present invention is for medical use, i.e. it is intended to be used to prevent and/or treat a disease.

As used herein,"treatment" or"treating" is an approach for obtaining beneficial or desired results including clinical results. For purposes of this invention, beneficial or desired clinical results include, but are not limited to, one or more of the following: alleviating one or more symptoms resulting from the disease, diminishing the extent of the disease, stabilizing the disease (e.g., preventing or delaying the worsening of the disease), preventing or delaying the spread of the disease, preventing or delaying the recurrence of the disease, delaying or slowing the progression of the disease, ameliorating the disease state, providing a remission (partial or total) of the disease, decreasing the dose of one or more other medications required to treat the disease, and/or prolonging survival. The use of the present invention contemplates any one or more of these aspects of treatment.

The term "prevent," and similar words such as "prevented", "preventing" etc., indicate an approach for preventing, inhibiting, or reducing the likelihood of the recurrence of, a disease or condition. It also refers to delaying the recurrence of a disease or condition or delaying the recurrence of the symptoms of a disease or condition. As used herein, "prevention" and similar terms also includes reducing the intensity, effect, symptoms and/or burden of a disease or condition prior to recurrence of the disease or condition.

In one embodiment, the liquid pharmaceutical composition of the present invention is used to prevent and/or treat an infection with a coronavirus binding to ACE2. Coronaviruses are enveloped viruses with a positive sense, single-stranded RNA genome and an icosahedral protein shell. The spike protein consisting of the S1 and S2 subunits forms a homotrimer which projects from the envelope and mediates the interaction with the target cells by binding to ACE2. Coronaviruses often cause respiratory diseases in humans and other mammalian as well as bird species. In humans, seven coronavirus strains are known: HCoV-OC43, HCoV-HKU1, HCoV-229E, HCoV-NL63, MERS-CoV, SARS-CoV and SARS-CoV-2. The first four coronavirus strains (HCoV-OC43, HCoV-HKU1, HCoV-229E, HCoV-NL63) cause only mild symptoms, whereas infection with MERS-CoV, SARS-CoV and SARS-CoV-2 may lead to severe, potentially life-threatening disease.

It has been shown that SARS-CoV, SARS-CoV-2 and HCoV-NL63 bind to ACE2 and use this binding to enter the target cells (Li et al. (2003) Nature 426(6965): 450-4; Hoffmann et al. (2020) Cell 181: 1-10; Hofmann et al. (2005) Proc Natl Acad Sci USA. 102(22):7988-93). Accordingly, the liquid pharmaceutical composition of the present invention can be used to treat and/or prevent infection with a coronavirus binding to ACE2, in particular infection with SARS-CoV, SARS-CoV-2 or HCoV-NL63. Further coronaviruses binding to ACE2 can be identified by inoculating cells expressing ACE2 either transiently or constitutively with pseudotyped VSV (vesicular stomatitis virus) expressing the coronavirus spike protein and a reporter protein and detecting the activity of the reporter protein after the inoculation period (see protocol in Hoffmann et al. (2020) Cell 181: 1-10). In one embodiment, the liquid pharmaceutical composition of the present invention is used to treat and/or prevent infection with a coronavirus binding to ACE2, wherein the coronavirus binding to ACE2 is not SARS-CoV.

In one embodiment, the liquid pharmaceutical composition of the present invention is used to treat and/or prevent infection with a coronavirus binding to ACE2, wherein the coronavirus binding to ACE2 is SARS-CoV-2 or a variant of SARS-CoV-2 comprising the amino acid substitution D614G and/or the amino acid substitution N439K. The variant of SARS-CoV-2 comprising the amino acid substitution D614G is described in Korber et al. (2020) Cell 182(4): 812-827 and the amino acid substitution N439K is described in Thomson et al. (https://doi.org/10.1101/2020.11.04.355842). In one embodiment, the liquid pharmaceutical composition of the present invention is used to treat and/or prevent infection with a coronavirus binding to ACE2, wherein the coronavirus binding to ACE2 is a variant of SARS-CoV-2 comprising the amino acid substitution D614G. The amino acid substitution D614G is caused by an A-to-G nucleotide mutation at position 23,403 in the Wuhan reference strain. The numbering of the amino acids in the variants refers to the numbering in the spike protein of SARS-CoV-2 according to SEQ ID NO: 13. Hence, a SARS-CoV-2 virus with the Spike protein according to SEQ ID NO: 13 is defined to be the wild-type SARS-CoV-2 from which any variants are derived.

In one embodiment, the fusion protein of the present invention is used to treat and/or prevent infection with a coronavirus binding to ACE2, wherein the coronavirus binding to ACE2 is a variant of SARS-CoV-2 comprising the amino acid substitution D614G and at least one additional amino acid substitution. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, N501Y, A570D, P681H, T716I, S982A and D1118H and comprising a deletion of amino acids 69, 70 and 145. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, Y453F, I692V and M1229I and comprising a deletion of amino acids 69 and 70. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, S13I, W152C and L452R. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, E484K and V1176F. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, L18F, T20N, P26S, D138Y, R190S, K417T, E484K, N501Y, H655Y, T1027I and V1176F. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, D80A, D215G, K417N, E484K, N501Y and A701V and comprising a deletion of amino acids 242, 243 and 244. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, L18F, D80A, D215G, K417N, E484K, N501Y and A701V and comprising a deletion of amino acids 242, 243 and 244. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions D614G, D80A, R246I, K417N, E484K, N501Y and A701V and comprising a deletion of amino acids 242, 243 and 244. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions E484Q and L452R. In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions E484K and D614G and comprising a deletion of amino acids 145 and 146.

In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions T19R, R158G, L452R, T478K, D614G, P681R and D950N and comprising a deletion of amino acids 156 and 157.

In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions K417N, N440K, G446S, S477N, T478K, E484A, Q493K, G496S, Q498R, N501Y, Y505H and P681H.

In one embodiment, the fusion protein of the present invention is used to treat and/or prevent infection with a coronavirus binding to ACE2, wherein the coronavirus binding to ACE2 is a variant of SARS-CoV-2 comprising the amino acid substitutions A67V, T95I, Y145D, L212I, G339D, S371L, S373P, S375F, Q493R, T547K, D614G, H655Y, N679K, N764K, D796Y, N856K, Q954H, N969K, L981F.

In one embodiment, the fusion protein of the present invention is used to treat and/or prevent infection with a coronavirus binding to ACE2, wherein the coronavirus binding to ACE2 is a variant of SARS-CoV-2 comprising the amino acid deletions N211, Y144, V143, G142, V70, H69.

The route of administration is in accordance with known and accepted methods, e.g., injection or infusion by subcutaneous, intravenous, intraperitoneal, intramuscular, intraarterial, intralesional or intraarticular routes. In another embodiment the liquid pharmaceutical composition of the present invention is to be administered intranasally, e.g. by means of a nasal spray, a nasal ointment or nasal drops. In another embodiment, the liquid pharmaceutical composition on protein of the present invention is administered by topical administration or by inhalation. Preferably, the liquid pharmaceutical composition of the present invention is administered by intravenous injection or infusion.

Dosages and desired drug concentrations of pharmaceutical compositions of the present invention may vary depending on the particular use envisioned. The determination of the appropriate dosage or route of administration is well within the skill of an ordinary artisan. Animal experiments provide reliable guidance for the determination of effective doses for human therapy. Interspecies scaling of effective doses can be performed following the principles laid down by Mordenti, J. and Chappell, W."The Use of Interspecies Scaling in Toxicokinetics," In Toxicokinetics and New Drug Development, Yacobi et al., Eds, Pergamon Press, New York 1989, pp.42-46.

In one embodiment, the liquid pharmaceutical composition of the present invention is administered intravenously at a dosage of 0.1 mg/kg body weight to 4 mg/kg body weight, such as a dosage of 0.1 mg/kg body weight, 0.2 mg/kg body weight, 0.3 mg/kg body weight, 0.4 mg/kg body weight, 0.5 mg/kg body weight, 0.6 mg/kg body weight, 0.7 mg/kg body weight, 0.8 mg/kg body weight, 0.9 mg/kg body weight, 1.0 mg/kg body weight, 1.1 mg/kg body weight, 1.2 mg/kg body weight, 1.3 mg/kg body weight, 1.4 mg/kg body weight, 1.5 mg/kg body weight, 1.6 mg/kg body weight, 1.7 mg/kg body weight, 1.8 mg/kg body weight, 1.9 mg/kg body weight, 2.0 mg/kg body weight, 2.1 mg/kg body weight, 2.2 mg/kg body weight, 2.3 mg/kg body weight, 2.4 mg/kg body weight, 2.5 mg/kg body weight, 2.6 mg/kg body weight, 2.7 mg/kg body weight, 2.8 mg/kg body weight, 2.9 mg/kg body weight, 3.0 mg/kg body weight, 3.1 mg/kg body weight, 3.2 mg/kg body weight, 3.3 mg/kg body weight, 3.4 mg/kg body weight, 3.5 mg/kg body weight, 3.6 mg/kg body weight, 3.7 mg/kg body weight, 3.8 mg/kg body weight, 3.9 mg/kg body weight or 4.0 mg/kg body weight, the mg referring to the mg of the ACE2 Fc fusion protein delivered with the liquid pharmaceutical composition. Preferably, the liquid pharmaceutical composition of the present invention is administered intravenously at a dosage of 2.5 mg/kg body weight, the mg referring to the mg of the ACE2 Fc fusion protein delivered with the liquid pharmaceutical composition. Preferably, the liquid pharmaceutical composition is diluted in a saline solution before administration. In one embodiment, the saline solution comprises sodium chloride at a total amount of 0.4 to 1.5 % (w/v), preferably 0.6 to 1.0 % (w/v), more preferably 0.9% (w/v).

In one embodiment, the liquid pharmaceutical composition of the present invention is administered intravenously at a dosage of 10 mg/kg body weight to 150 mg/kg body weight, such as a dosage of 10 mg/kg body weight, 15 mg/kg body weight, 20 mg/kg body weight, 25 mg/kg body weight, 30 mg/kg body weight, 35 mg/kg body weight, 40 mg/kg body weight, 45 mg/kg body weight, 50 mg/kg body weight, 55 mg/kg body weight, 60 mg/kg body weight, 65 mg/kg body weight, 70 mg/kg body weight, 75 mg/kg body weight, 80 mg/kg body weight, 85 mg/kg body weight, 90 mg/kg body weight, 95 mg/kg body weight, 100 mg/kg body weight, 105 mg/kg body weight, 110 mg/kg body weight, 115 mg/kg body weight, 120 mg/kg body weight, 125 mg/kg body weight, 130 mg/kg body weight, 135 mg/kg body weight, 140 mg/kg body weight, 145 mg/kg body weight or 150 mg/kg body weight, the mg referring to the mg of the ACE2 Fc fusion protein delivered with the liquid pharmaceutical composition. Preferably, the liquid pharmaceutical composition of the present invention is administered intravenously at a dosage of 15 mg/kg body weight, the mg referring to the mg of the ACE2 Fc fusion protein delivered with the liquid pharmaceutical composition. Preferably, the liquid pharmaceutical composition is diluted in a saline solution before administration. In one embodiment, the saline solution comprises sodium chloride at a total amount of 0.4 to 1.5 % (w/v), preferably 0.6 to 1.0 % (w/v), more preferably 0.9% (w/v).

The liquid pharmaceutical composition may be administered once per day, twice per day, three times per day, every other day, once per week or once every two weeks.

The liquid pharmaceutical composition may be administered for a period of three days, four days, five days, six days, seven days, eight days, nine days or ten days.

By administering the liquid pharmaceutical composition of the present invention, the infection with a coronavirus and in particular with SARS-CoV-2 is treated, i.e. at least one of the symptoms of an infection with SARS-CoV-2 is reduced or abolished. Symptoms of an infection with SARS-CoV-2 include coughing, shortness of breath, difficulty breathing, fever, chills, tiredness, muscle aches, sore throat, headache, chest pain and loss of smell and/or taste. In one embodiment, by the administration of the fusion protein of the present invention the fever caused by infection with SARS-CoV-2 is reduced. In one embodiment, the administration of the liquid pharmaceutical composition of the present invention to a subject reduces the risk that the subject experiences a severe course of infection with SARS-CoV-2. In one embodiment, the administration of the liquid pharmaceutical composition of the present invention to a subject reduces the risk that the subject experiences multi-organ failure, acute respiratory distress syndrome (ARDS) or pneumonia. In one embodiment, the administration of the liquid pharmaceutical composition of the present invention to a subject reduces the risk that the subject experiences long-term effects of the infection with SARS-CoV-2 such as lung damage, neurological disorders, dermatological disorders and cardiovascular disease. In one embodiment, the administration of the liquid pharmaceutical composition of the present invention to a subject reduces the concentration of the cytokines IL6 and/or IL8 in the blood. In one embodiment, the administration of the fusion protein of the present invention to a subject reduces the concentration of SARS-CoV-2 virus particles in the blood. In one embodiment, the administration of the fusion protein of the present invention to a subject stimulates the production of antiviral antibodies. In one embodiment, the administration of the fusion protein of the present invention to a subject stimulates the production of antiviral IgA and/or IgG antibodies.

In one embodiment, the fusion protein of the present invention is administered to a subject suffering from a severe infection with SARS-CoV-2. In one embodiment, the fusion protein of the present invention is administered to a subject infected with SARS-CoV-2 and requiring artificial ventilation. In one embodiment, the fusion protein of the present invention is administered to a subject infected with SARS-CoV-2 and requiring extracorporeal membrane oxygenation (ECMO).

By administering the fusion protein of the present invention, the infection with a coronavirus and in particular with SARS-CoV-2 is prevented, i.e. the treated subject does not develop symptoms of an infection with SARS-CoV-2.

In one embodiment, the fusion protein of the present invention is administered to a subject which has been in contact with a subject infected with SARS-CoV-2. Subjects which have been in contact with a subject infected with SARS-CoV-2 can be identified by use of a "Corona warning app" installed on the smartphone.

In one embodiment, the fusion protein of the present invention is administered to a subject for which a test with a throat swab of said subject indicates that it is infected with SARS-CoV-2, but which has not developed any symptoms of an infection with SARS-CoV-2.

In the treatment or prevention of an infection with a coronavirus binding to ACE2 and in particular SARS-CoV-2 the fusion protein of the present invention may be combined with a known anti-viral agent. Anti-viral agents are medicaments used to treat viral infections and include both specific anti-viral agents and broad-spectrum viral agents. Suitable anti-viral agents include, but are not limited to, nucleoside analoga, inhibitors of viral protease, inhibitors of viral polymerase, blockers of virus entry into the cell, Janus kinase inhibitors, but also inhibitors of inflammatory mediators.

In specific embodiments, the anti-viral agent is selected from the group consisting of remdesivir, arbidol HCl, ritonavir, lopinavir, darunavir, ribavirin, chloroquin and derivatives thereof such as hydroxychloroquin, nitazoxanide, camostat mesilate, anti-IL6 and anti-IL6 receptor antibodies such as tocilizumab, siltuximab and sarilumab and baricitinib phosphate.

In the treatment or prevention of an infection with SARS-CoV-2 the pharmaceutical composition of the present invention may further contain or be combined with an anti-SARS-CoV-2 monoclonal antibody. Anti-SARS-CoV2 monoclonal antibodies include, but are not limited to, bamlanivimab (LY-CoV555 20; LY3819253;) developed by Eli Lilly and Company, etesevimab (LY-3832479; LY-COV016; JS-016; NP-005), REGN-COV2 which is a cocktail of REGN10933 (casivirimab) and REGN10987 (imdevimab) and which is developed by Regeneron, sotrovimab (VIR-7831; GSK4182136;) developed by Vir Biotechnology and GlaxoSmithKline, CT-P59 developed by Celltrion, AZD 7442 which is a combination of antibodies AZD8895 and AZD1061 and which is developed by Astra Zeneca, JS016 developed by Junshi Biosciences, TY027 developed 25 by Tychan Pte Ltd, BRII-96 and BRII-98 developed by Brii Biosciences, SCTA01 developed by Sinocelltech Ltd, ADM03820 developed by Ology Bioservices, BI767551 developed by Boehringer Ingelheim and others and COR-101 developed by Corat Therapeutics, Paxlovid (PF-07321332; ritonavir) developed by Pfizer Inc.

Apart from its function in binding coronaviruses, ACE2 has also been implicated in several disorders and diseases such as hypertension (including high blood pressure), congestive heart failure, chronic heart failure, acute heart failure, contractile heart failure, myocardial infarction, arteriosclerosis, kidney failure, renal failure, Acute Respiratory Distress Syndrome (ARDS), Acute Lung Injury (ALI), chronic obstructive pulmonary disease (COPD), pulmonary hypertension, renal fibrosis, chronic renal failure, acute renal failure, acute kidney injury, inflammatory bowel disease and multi-organ dysfunction syndrome. Hence, the fusion protein of the present invention can also be used in the treatment of these disorders and diseases.

The pharmaceutical compositions may be supplied in a vial or in a pre-filled syringe. The pharmaceutical compositions may be administered by intravenous infusion, e.g. over a period of 30 minutes or less. The pharmaceutical compositions may be administered by intravenous infusion, e.g. over a period of 30 minutes to 1 hour, preferably 1 hour.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

The detailed description is merely exemplary in nature and is not intended to limit application and uses. The following examples further illustrate the present invention without, however, limiting the scope of the invention thereto. Various changes and modifications can be made by those skilled in the art on the basis of the description of the invention, and such changes and modifications are also included in the present invention.

### EXAMPLES

### Example 1: Formulations selected for stability study

A multimeric ACE2-lgM-Fc fusion construct is transferred into 12 different formulations according to Table 1 by dialysis or UF/DF with a target concentration of 20 mg/mL or 40 mg/mL of the tested ACE2-IgM-Fc fusion construct and evaluated during a stability study at different temperatures and stress conditions for best stabilizing effects regarding physicochemical stability of the oligomeric structure and its binding/ potency.

**Table 1: Detailed information of formulations prepared within this study for stabilizing ACE2-lgM-Fc fusion construct at strengths of 20 mg/mL and 40 mg/mL**

| **No.** | **Buffer** | **Stabilizer I** | **Stabilizer II** | **Stabilizer III** | **Stabilizer IV** | **Surfactant** | **pH** |
|---|---|---|---|---|---|---|---|
| **(1)** | 10 mM acetic acid | 250 mM trehalose dihydrate | n.a. | n.a. | n.a. | 0.2 mg/mL polysorbate 20 | 5.4 |
| **(2)** | 10 mM acetic acid | 250 mM trehalose dihydrate | 30 mM L-arginine | n.a. | n.a. | 0.2 mg/mL polysorbate 20 | 5.4 |
| **(3)** | 10 mM acetic acid | 250 mM trehalose dihydrate | n.a. | 50 mM NaCl | n.a. | 0.2 mg/mL polysorbate 20 | 5.4 |
| **(4)** | 10 mM acetic acid | 250 mM trehalose dihydrate | 30 mM L-arginine | 50 mM NaCl | n.a. | 0.2 mg/mL polysorbate 20 | 5.4 |
| **(5)** | 10 mM acetic acid | 250 mM trehalose dihydrate | n.a. | n.a. | 5 mM L-methionine | 0.2 mg/mL polysorbate 20 | 5.4 |
| **(6)** | 10 mM acetic acid | 250 mM trehalose dihydrate | 30 mM L-arginine | 50 mM NaCl | 5 mM L-methionine | 0.2 mg/mL polysorbate 20 | 5.4 |
| **(7)** | 10 mM acetic acid | 250 mM trehalose dihydrate | n.a. | n.a. | n.a. | 0.2 mg/mL polysorbate 20 | 5.8 |
| **(8)** | 10 mM acetic acid | 250 mM trehalose dihydrate | 30 mM L-arginine | n.a. | n.a. | 0.2 mg/mL polysorbate 20 | 5.8 |
| **(9)** | 10 mM acetic acid | 250 mM trehalose dihydrate | n.a. | 50 mM NaCl | n.a. | 0.2 mg/mL polysorbate 20 | 5.8 |
| **(10)** | 10 mM acetic acid | 250 mM trehalose dihydrate | 30 mM L-arginine | 50 mM NaCl | n.a. | 0.2 mg/mL polysorbate 20 | 5.8 |
| **(11)** | 10 mM acetic acid | 250 mM trehalose dihydrate | n.a. | n.a. | 5 mM L-methionine | 0.2 mg/mL polysorbate 20 | 5.8 |
| **(12)** | 10 mM acetic acid | 250 mM trehalose dihydrate | 30 mM L-arginine | 50 mM NaCl | 5 mM L-methionine | 0.2 mg/mL polysorbate 20 | 5.8 |

If necessary, the pH is adjusted to the target pH by using HCl or NaOH.

Samples are stored for up to 12 months under the conditions shown in Table 2. In addition, samples are subjected to five freeze/ thaw cycles.

**Table 2: Storage stability study of ACE2-lgM-FC fusion construct**

| **Stress Type/ Time Point (months)** | **t0** | **1** | **2** | **3** | **6** | **12** |
|---|---|---|---|---|---|---|
| **5°C** | X | | | X | X | X |
| **25°C ± 2°C / 60 % RH ± 5 % RH** | | X | | X | X | |
| **40°C ± 2°C / 75 % RH ± 5 % RH** | | X | X | X | | |
| **Freeze/ thaw (-80°C / +25 °C)** | | 5 cycles | | | | |

Protein stability is determined during the stability study by size exclusion chromatography with using multi-angle light scattering detection for the content of ACE2-lgM-Fc multimers (here a hexameric structure of ICE2-IgM-FC dimer), higher molecular weight species (HMWS) and dimeric or smaller structures.

With SDS-cGE non-reducing or SDS-PAGE non reducing the generation of low molecular weight species (LMWS) is quantified. Melting points indicating stability against unfolding is analyzed by differential scanning fluorometry (DSF).

Peptide mapping is used to quantify modifications like oxidations, deamidation and glycation.

Ion exchange chromatography (IEX-HPLC) as well as imaged capillary isoelectric focusing (icIEF) detects modifications leading to charge heterogeneities.

The protein concentration of the samples is determined by UV-VIS spectroscopy.

Binding of the ACE2-lgM-FC construct in the different formulations during stability studies is determined by surface plasmon resonance (SPR).

The inhibition of SARS-CoV-2 RBD binding to ACE2 is quantified by ELISA.

Some embodiments of the present invention relate to:
1. A liquid pharmaceutical composition comprising:
   (a) a fusion protein comprising a first part comprising a fragment of human ACE2 or a variant of said fragment, said human ACE2 having the amino acid sequence according to SEQ ID NO: 1, and a second part comprising the Fc portion of a human IgM or a fragment or variant of the Fc portion of human IgM; and
   (b) a buffer having a pH of 5.2 to 6.0
2. The liquid pharmaceutical composition according to item 1, wherein the buffer is selected from the group consisting of acetate buffer, histidine buffer, phosphate buffer, citrate buffer and succinate buffer.
3. The liquid pharmaceutical composition according to item 1 or 2, wherein the buffer is present in a concentration of 5 mM to 60 mM.
4. A liquid pharmaceutical composition comprising:
   (a) a fusion protein comprising a first part comprising a fragment of human ACE2 or a variant of said fragment, said human ACE2 having the amino acid sequence according to SEQ ID NO: 1, and a second part comprising the Fc portion of a human IgM or a fragment or variant of the Fc portion of human IgM; and
   (b) an acetate buffer having a pH of 5.2 to 6.0.
5. The liquid pharmaceutical composition according to item 4, wherein the acetate buffer is present in a concentration of 5 mM to 60 mM.
6. The liquid pharmaceutical composition according to any one of the preceding items, further comprising a sugar or a sugar alcohol.
7. The liquid pharmaceutical composition according to item 6, wherein the sugar is selected from trehalose and sucrose.
8. The liquid pharmaceutical composition according to item 6 or 7, wherein the sugar is present in a concentration of 100 mM to 300 mM.
9. The liquid pharmaceutical composition according to any one of the preceding items, further comprising a non-ionic surfactant.
10. The liquid pharmaceutical composition according to item 9, wherein the non-ionic surfactant is selected from polysorbate 20 and polysorbate 80.
11. The liquid pharmaceutical composition according to item 9 or 10, wherein the non-ionic surfactant is present in a concentration of 0.01 % (w/v) to 0.2% (w/v).
12. The liquid pharmaceutical composition according to any one of the preceding items, further comprising an inorganic salt.
13. The liquid pharmaceutical composition according to item 12, wherein the inorganic salt is sodium chloride.
14. The liquid pharmaceutical composition according to item 12 or 13, wherein the inorganic salt is present in a concentration of 30 mM to 150 mM.
15. The liquid pharmaceutical composition according to any one of the preceding items, further comprising one or more amino acids.
16. The liquid pharmaceutical composition according to item 15, wherein the one or more amino acids are L-arginine and/or L-methionine.
17. The liquid pharmaceutical composition according to item 15 or 16, wherein the one or more amino acids are present in a concentration of 1 mM to 50 mM.
18. The liquid pharmaceutical composition according to any one of the preceding items, wherein the Fc portion of human IgM has the amino acid sequence according to SEQ ID NO: 4.
19. The liquid pharmaceutical composition according to any one of items 1 to 17, wherein the fragment of the Fc portion of human IgM consists of the amino acid sequence according to SEQ ID No 7.
20. The liquid pharmaceutical composition according to any one of the preceding items, wherein the fragment of human ACE2 consists of the amino acid sequence according to SEQ ID NO: 2.
21. The liquid pharmaceutical composition according to any one of items 1 to 19, wherein the fragment of human ACE2 is the extracellular domain of ACE2 consisting of the amino acid sequence according to SEQ ID NO: 3.
22. The liquid pharmaceutical composition according to any one of the preceding items, wherein the first part and the second part of the fusion protein are linked by a linker sequence.
23. The liquid pharmaceutical composition according to item 22, wherein the linker sequence consists of glycine and serine residues.
24. The liquid pharmaceutical composition according to item 22 or 23, wherein the linker sequence has the amino acid sequence according to SEQ ID NO: 5.
25. The liquid pharmaceutical composition according to any one of the preceding items, wherein the fusion protein further comprises a signal peptide.
26. The liquid pharmaceutical composition according to item 25, wherein the signal peptide comprises the amino acid sequence according to SEQ ID NO: 6.
27. The liquid pharmaceutical composition according to any one of the preceding items, wherein the variant of the human ACE2 fragment is an enzymatically inactive variant of human ACE2.
28. The liquid pharmaceutical composition according to item 27, wherein the enzymatically inactive variant of human ACE2 comprises a H374N and a H378N mutation, the numbering referring to SEQ ID NO: 1.
29. The liquid pharmaceutical composition according to any one of items 1 to 18 and 20 to 26, wherein the fusion protein has the amino acid sequence according to SEQ ID NO: 8.
30. The liquid pharmaceutical composition according to any one of items 1 to 17 and 19 to 26, wherein the fusion protein has the amino acid sequence according to SEQ ID NO: 9.
31. The liquid pharmaceutical composition according to any one of the preceding items, wherein the fusion protein is present as a multimer formed by at least two molecules of the fusion protein characterized as in any one of items 1, 4 and 18 to 30.
32. The liquid pharmaceutical composition according to any one of the preceding items, wherein the fusion protein is present in a concentration of 1-60 mg/ml.
33. A liquid pharmaceutical composition comprising:
   (a) a fusion protein comprising a first part comprising a fragment of human ACE2 or a variant of said fragment, said human ACE2 having the amino acid sequence according to SEQ ID NO: 1, and a second part comprising the Fc portion of a human IgM or a fragment or variant of the Fc portion of human IgM; and
   (b) an acetate buffer having a pH of 5.4 to 5.8;
   (c) polysorbate 20 or polysorbate 80;
   (d) trehalose or sucrose; and
   (e) optionally, one or more stabilizers selected from the group consisting of L-arginine, L-methionine and sodium chloride.
34. The liquid pharmaceutical composition according to item 33, wherein the acetate buffer is present in a concentration of 5 mM to 60 mM.
35. The liquid pharmaceutical composition according to item 33 or 34, wherein the composition comprises trehalose which is present in a concentration of 100 mM to 250 mM.
36. The liquid pharmaceutical composition according to any one of items 33 to 35, wherein the composition comprises polysorbate 20 which is present in a concentration of 0.01 % (w/v) to 0.2% (w/v).
37. The liquid pharmaceutical composition according to any one of items 33 to 36, further comprising sodium chloride.
38. The liquid pharmaceutical composition according to item 37, wherein the sodium chloride is present in a concentration of 30 mM to 150 mM.
39. The liquid pharmaceutical composition according to any one of items 33 to 38, wherein the composition comprises L-arginine which is present in a concentration of 10 mM to 50 mM.
40. The liquid pharmaceutical composition according to any one of items 33 to 39, wherein the composition comprises L-methionine which is present in a concentration of 1 mM to 20 mM.
41. The liquid pharmaceutical composition according to any one of items 33 to 40, wherein the fragment of human ACE2 consists of the amino acid sequence according to SEQ ID NO: 2.
42. The liquid pharmaceutical composition according to any one of items 33 to 40, wherein the fragment of human ACE2 is the extracellular domain of ACE2 consisting of the amino acid sequence according to SEQ ID NO: 3.
43. The liquid pharmaceutical composition according to any one of items 33 to 42, wherein the Fc portion of human IgM has the amino acid sequence according to SEQ ID NO: 4.
44. The liquid pharmaceutical composition according to any one of items 33 to 42, wherein the fragment of the Fc portion of human IgM consists of the amino acid sequence according to SEQ ID No 7.
45. The liquid pharmaceutical composition according to any one of items 33 to 44, wherein the first part and the second part of the fusion protein are linked by a linker sequence.
46. The liquid pharmaceutical composition according to item 45, wherein the linker sequence consists of glycine and serine residues.
47. The liquid pharmaceutical composition according to item 45 or 46, wherein the linker sequence has the amino acid sequence according to SEQ ID NO: 5.
48. The liquid pharmaceutical composition according to any one of items 33 to 47, wherein the fusion protein further comprises a signal peptide.
49. The liquid pharmaceutical composition according to item 48, wherein the signal peptide comprises the amino acid sequence according to SEQ ID NO: 6.
50. The liquid pharmaceutical composition according to any one of items 33 to 43 and 45 to 49, wherein the fusion protein has the amino acid sequence according to SEQ ID NO: 8.
51. The liquid pharmaceutical composition according to any one of items 33 to 42 and 44 to 49, wherein the fusion protein has the amino acid sequence according to SEQ ID NO: 9.
52. The liquid pharmaceutical composition according to any one of items 33 to 49, wherein the variant of the human ACE2 fragment is an enzymatically inactive variant of human ACE2.
53. The liquid pharmaceutical composition according to item 52, wherein the enzymatically inactive variant of human ACE2 comprises a H374N and a H378N mutation, the numbering referring to SEQ ID NO: 1.
54. The liquid pharmaceutical composition according to any one of items 33 to 53, wherein the fusion protein is present as a multimer formed by at least two molecules of the fusion protein characterized as in any one of items 33 and 41 to 53.
55. The liquid pharmaceutical composition according to any one of items 33 to 54, wherein the fusion protein is present in a concentration of 1-60 mg/ml.
56. A liquid pharmaceutical composition comprising a fusion protein according to SEQ ID NO: 8 or 9, acetate buffer having a pH of 5.4, polysorbate 20, trehalose, and water for injection.
57. The liquid pharmaceutical composition according to item 56, comprising 1 to 60 mg/ml of a fusion protein according to SEQ ID NO: 8 or 9, 10 mM acetate buffer having a pH of 5.4, 0.02% (w/v) polysorbate 20, 250 mM trehalose and water for injection.
58. A liquid pharmaceutical composition comprising a fusion protein according to SEQ ID NO: 8 or 9, acetate buffer having a pH of 5.4, polysorbate 20, trehalose, sodium chloride and water for injection.
59. The liquid pharmaceutical composition according to item 58, comprising 1 to 60 mg/ml of a fusion protein according to SEQ ID NO: 8 or 9, 10 mM acetate buffer having a pH of 5.4, 0.02% (w/v) polysorbate 20, 250 mM trehalose, 50 mM sodium chloride and water for injection.
60. The liquid pharmaceutical composition according to item 58 or 59, further comprising L-arginine.
61. The liquid pharmaceutical composition according to item 60, wherein the concentration of L-arginine is 30 mM.
62. The liquid pharmaceutical composition according to any one of items to 58 to 61, further comprising L-methionine.
63. The liquid pharmaceutical composition according to item 62, wherein the concentration of L-methionine is 5 mM.
64. A liquid pharmaceutical composition consisting of a fusion protein according to SEQ ID NO: 8 or 9, acetate buffer having a pH of 5.4, polysorbate 20, trehalose and water for injection.
65. The liquid pharmaceutical composition according to item 64, consisting of 1 to 60 mg/ml of a fusion protein according to SEQ ID NO: 8 or 9, 10 mM acetate buffer having a pH of 5.4, 0.02% (w/v) polysorbate 20, 250 mM trehalose and water for injection.
66. A liquid pharmaceutical composition consisting of a fusion protein according to SEQ ID NO: 8 or 9, acetate buffer having a pH of 5.4, polysorbate 20, trehalose and water for injection.
67. The liquid pharmaceutical composition according to item 66, consisting of 1 to 60 mg/ml of a fusion protein according to SEQ ID NO: 8 or 9, 10 mM acetate buffer having a pH of 5.4, 0.02% (w/v) polysorbate 20, 250 mM trehalose and water for injection.
68. A liquid pharmaceutical composition consisting of a fusion protein according to SEQ ID NO: 8 or 9, acetate buffer having a pH of 5.4, polysorbate 20, trehalose, sodium chloride and water for injection.
69. The liquid pharmaceutical composition according to item 68, consisting of 1 to 60 mg/ml of a fusion protein according to SEQ ID NO: 8 or 9, 10 mM acetate buffer having a pH of 5.4, 0.02% (w/v) polysorbate 20, 250 mM trehalose, 50 mM sodium chloride and water for injection.
70. A liquid pharmaceutical composition consisting of a fusion protein according to SEQ ID NO: 8 or 9, acetate buffer having a pH of 5.4, polysorbate 20, trehalose, L-arginine and water for injection.
71. The liquid pharmaceutical composition according to item 70, consisting of 1 to 60 mg/ml of a fusion protein according to SEQ ID NO: 8 or 9, 10 mM acetate buffer having a pH of 5.4, 0.02% (w/v) polysorbate 20, 250 mM trehalose, 30 mM L-arginine and water for injection.
72. A liquid pharmaceutical composition consisting of a fusion protein according to SEQ ID NO: 8 or 9, acetate buffer having a pH of 5.4, polysorbate 20, trehalose, sodium chloride, L-arginine and water for injection.
73. The liquid pharmaceutical composition according to item 72, consisting of 1 to 60 mg/ml of a fusion protein according to SEQ ID NO: 8 or 9, 10 mM acetate buffer having a pH of 5.4, 0.02% (w/v) polysorbate 20, 250 mM trehalose, 50 mM sodium chloride, 30 mM L-arginine and water for injection.
74. A liquid pharmaceutical composition consisting of a fusion protein according to SEQ ID NO: 8 or 9, acetate buffer having a pH of 5.4, polysorbate 20, trehalose, L-methionine and water for injection.
75. The liquid pharmaceutical composition according to item 74, consisting of 1 to 60 mg/ml of a fusion protein according to SEQ ID NO: 8 or 9, 10 mM acetate buffer having a pH of 5.4, 0.02% (w/v) polysorbate 20, 250 mM trehalose, 5 mM L-methionine and water for injection.
76. A liquid pharmaceutical composition consisting of a fusion protein according to SEQ ID NO: 8 or 9, acetate buffer having a pH of 5.4, polysorbate 20, trehalose, sodium chloride, L-arginine, L-methionine and water for injection.
77. The liquid pharmaceutical composition according to item 76, consisting of 1 to 60 mg/ml of a fusion protein according to SEQ ID NO: 8 or 9, 10 mM acetate buffer having a pH of 5.4, 0.02% (w/v) polysorbate 20, 250 mM trehalose, 50 mM sodium chloride, 30 mM L-arginine, 5 mM L-methionine and water for injection.
78. A liquid pharmaceutical composition comprising a fusion protein according to SEQ ID NO: 8 or 9, acetate buffer having a pH of 5.8, polysorbate 20, trehalose, and water for injection.
79. The liquid pharmaceutical composition according to item 78, comprising 1 to 60 mg/ml of a fusion protein according to SEQ ID NO: 8 or 9, 10 mM acetate buffer having a pH of 5.8, 0.02% (w/v) polysorbate 20, 250 mM trehalose and water for injection.
80. A liquid pharmaceutical composition comprising a fusion protein according to SEQ ID NO: 8 or 9, acetate buffer having a pH of 5.8, polysorbate 20, trehalose, sodium chloride and water for injection.
81. The liquid pharmaceutical composition according to item 80, comprising 1 to 60 mg/ml of a fusion protein according to SEQ ID NO: 8 or 9, 10 mM acetate buffer having a pH of 5.8, 0.02% (w/v) polysorbate 20, 250 mM trehalose, 50 mM sodium chloride and water for injection.
82. The liquid pharmaceutical composition according to item 80 to 81, further comprising L-arginine.
83. The liquid pharmaceutical composition according to item 82, wherein the concentration of L-arginine is 30 mM.
84. The liquid pharmaceutical composition according to any one of items to 80 to 83, further comprising L-methionine.
85. The liquid pharmaceutical composition according to item 84, wherein the concentration of L-methionine is 5 mM.
86. A liquid pharmaceutical composition consisting of a fusion protein according to SEQ ID NO: 8 or 9, acetate buffer having a pH of 5.8, polysorbate 20, trehalose and water for injection.
87. The liquid pharmaceutical composition according to item 86, consisting of 1 to 60 mg/ml of a fusion protein according to SEQ ID NO: 8 or 9, 10 mM acetate buffer having a pH of 5.8, 0.02% (w/v) polysorbate 20, 250 mM trehalose and water for injection.
88. A liquid pharmaceutical composition consisting of a fusion protein according to SEQ ID NO: 8 or 9, acetate buffer having a pH of 5.8, polysorbate 20, trehalose and water for injection.
89. The liquid pharmaceutical composition according to item 88, consisting of 1 to 60 mg/ml of a fusion protein according to SEQ ID NO: 8 or 9, 10 mM acetate buffer having a pH of 5.8, 0.02% (w/v) polysorbate 20, 250 mM trehalose and water for injection.
90. A liquid pharmaceutical composition consisting of a fusion protein according to SEQ ID NO: 8 or 9, acetate buffer having a pH of 5.8, polysorbate 20, trehalose, sodium chloride and water for injection.
91. The liquid pharmaceutical composition according to item 90, consisting of 1 to 60 mg/ml of a fusion protein according to SEQ ID NO: 8 or 9, 10 mM acetate buffer having a pH of 5.8, 0.02% (w/v) polysorbate 20, 250 mM trehalose, 50 mM sodium chloride and water for injection.
92. A liquid pharmaceutical composition consisting of a fusion protein according to SEQ ID NO: 8 or 9, acetate buffer having a pH of 5.8, polysorbate 20, trehalose, L-arginine and water for injection.
93. The liquid pharmaceutical composition according to item 92, consisting of 1 to 60 mg/ml of a fusion protein according to SEQ ID NO: 8 or 9, 10 mM acetate buffer having a pH of 5.8, 0.02% (w/v) polysorbate 20, 250 mM trehalose, 30 mM L-arginine and water for injection.
94. A liquid pharmaceutical composition consisting of a fusion protein according to SEQ ID NO: 8 or 9, acetate buffer having a pH of 5.8, polysorbate 20, trehalose, sodium chloride, L-arginine and water for injection.
95. The liquid pharmaceutical composition according to item 94, consisting of 1 to 60 mg/ml of a fusion protein according to SEQ ID NO: 8 or 9, 10 mM acetate buffer having a pH of 5.8, 0.02% (w/v) polysorbate 20, 250 mM trehalose, 50 mM sodium chloride, 30 mM L-arginine and water for injection.
96. A liquid pharmaceutical composition consisting of a fusion protein according to SEQ ID NO: 8 or 9, acetate buffer having a pH of 5.8, polysorbate 20, trehalose, L-methionine and water for injection.
97. The liquid pharmaceutical composition according to item 96, consisting of 1 to 60 mg/ml of a fusion protein according to SEQ ID NO: 8 or 9, 10 mM acetate buffer having a pH of 5.8, 0.02% (w/v) polysorbate 20, 250 mM trehalose, 5 mM L-methionine and water for injection.
98. A liquid pharmaceutical composition consisting of a fusion protein according to SEQ ID NO: 8 or 9, acetate buffer having a pH of 5.8, polysorbate 20, trehalose, sodium chloride, L-arginine, L-methionine and water for injection.
99. The liquid pharmaceutical composition according to item 98, consisting of 1 to 60 mg/ml of a fusion protein according to SEQ ID NO: 8 or 9, 10 mM acetate buffer having a pH of 5.8, 0.02% (w/v) polysorbate 20, 250 mM trehalose, 50 mM sodium chloride, 30 mM L-arginine, 5 mM L-methionine and water for injection.
100. The liquid pharmaceutical composition according to any one of the items 56 to 99, wherein the fusion protein is present as a multimer formed by at least two molecules of the fusion protein characterized as in any one of items 56 to 99.
101. The liquid pharmaceutical composition according to any one of the preceding items for use in preventing and/or treating an infection with a coronavirus binding to ACE2.
102. The liquid pharmaceutical composition for use according to item 101, wherein the coronavirus binding to ACE2 is selected from the group consisting of SARS, SARS-CoV2 and NL63, preferably it is SARS-CoV2.
103. The liquid pharmaceutical composition for use according to item 101 or 102, wherein the liquid pharmaceutical composition is to be administered in combination with an anti-viral agent.
104. The liquid pharmaceutical composition for use according to item 103, wherein the anti-viral agent is selected from the group consisting of remdesivir, arbidol HCl, ritonavir, lopinavir, darunavir, ribavirin, chloroquin and derivatives thereof, nitazoxanide, camostat mesilate, tocilizumab, siltuximab, sarilumab, paxlovid and baricitinib phosphate.
105. The liquid pharmaceutical composition according to any one of items 1 to 100 for use in treating hypertension (including high blood pressure), congestive heart failure, chronic heart failure, acute heart failure, contractile heart failure, myocardial infarction, arteriosclerosis, kidney failure, renal failure, Acute Respiratory Distress Syndrome (ARDS), Acute Lung Injury (ALI), chronic obstructive pulmonary disease (COPD), pulmonary hypertension, renal fibrosis, chronic renal failure, acute renal failure, acute kidney injury, inflammatory bowel disease and multi-organ dysfunction syndrome.

## Claims

1. A liquid pharmaceutical composition comprising:
(a) a fusion protein comprising a first part comprising a fragment of human ACE2 or a variant of said fragment, said human ACE2 having the amino acid sequence according to SEQ ID NO: 1, and a second part comprising the Fc portion of a human IgM or a fragment or variant of the Fc portion of human IgM; and
(b) a buffer having a pH of 5.2 to 6.0.

2. The liquid pharmaceutical composition according to claim 1, wherein the buffer is selected from the group consisting of acetate buffer, histidine buffer, phosphate buffer, citrate buffer, and succinate buffer, preferably wherein the buffer is present in a concentration of 5 mM to 60 mM.

3. The liquid pharmaceutical composition according to claim 1 or 2, further comprising a sugar or a sugar alcohol, preferably wherein the sugar or sugar alcohol is selected from trehalose, sucrose and mannitol and/or wherein the sugar or sugar alcohol is present in a concentration of 100 mM to 300 mM.

4. The liquid pharmaceutical composition according to any one of the preceding claims, further comprising a non-ionic surfactant, preferably wherein the non-ionic surfactant is selected from polysorbate 20 and polysorbate 80 and/or wherein the non-ionic surfactant is present in a concentration of 0.01 % (w/v) to 0.2% (w/v).

5. The liquid pharmaceutical composition according to any one of the preceding claims, further comprising an inorganic salt, preferably wherein the inorganic salt is sodium chloride and/or wherein the inorganic salt is present in a concentration of 30 mM to 150 mM.

6. The liquid pharmaceutical composition according to any one of the preceding claims, further comprising one or more amino acids, preferably wherein the one or more amino acids are L-arginine and/or L-methionine and/or wherein the one or more amino acids are present in a concentration of 1 mM to 50 mM.

7. The liquid pharmaceutical composition according to any one of the preceding claims, wherein the fragment of human ACE2 consists of the amino acid sequence according to SEQ ID NO: 2 or wherein the fragment of human ACE2 is the extracellular domain of ACE2 consisting of the amino acid sequence according to SEQ ID NO: 3.

8. The liquid pharmaceutical composition according to any one of the preceding claims, wherein the Fc portion of human IgM has the amino acid sequence according to SEQ ID NO: 4 or wherein the fragment of the Fc portion of human IgM consists of the amino acid sequence according to SEQ ID No 7..

9. The liquid pharmaceutical composition according to any one of the preceding claims, wherein the fusion protein has the amino acid sequence according to SEQ ID NO: 8 or 9.

10. The liquid pharmaceutical composition according to any one of claims 1 to 8, wherein the variant of the human ACE2 fragment is an enzymatically inactive variant of human ACE2, preferably wherein the enzymatically inactive variant of human ACE2 comprises a H374N and a H378N mutation, the numbering referring to SEQ ID NO: 1.

11. The liquid pharmaceutical composition according to any one of the preceding claims, wherein the fusion protein is present as a multimer formed by at least two molecules of the fusion protein characterized as in any one of claims 1 and 7 to 10.

12. A liquid pharmaceutical composition comprising:
(a) a fusion protein comprising a first part comprising a fragment of human ACE2 or a variant of said fragment, said human ACE2 having the amino acid sequence according to SEQ ID NO: 1, and a second part comprising the Fc portion of a human IgM or a fragment or variant of the Fc portion of human IgM; and
(b) an acetate buffer having a pH of 5.4 to 5.8;
(c) polysorbate 20 or polysorbate 80;
(d) trehalose or sucrose; and
(e) optionally, one or more stabilizers selected from the group consisting of L-arginine, L-methionine and sodium chloride.

13. The liquid pharmaceutical composition according to any one of the preceding claims, wherein the fusion protein is present in a concentration of 1-60 mg/ml.

14. The liquid pharmaceutical composition according to any one of the preceding claims for use in preventing and/or treating an infection with a coronavirus binding to ACE2, preferably wherein the coronavirus binding to ACE2 is selected from the group consisting of SARS, SARS-CoV2 and NL63, preferably it is SARS-CoV2.
